# EUROPEAN PATENT APPLICATION

(11) **EP 4 606 382 A1**
(43) Date of publication of application: **27.08.2025**
(21) Application number: 24382177.4
(22) Date of filing: 20.02.2024
(51) Int. Cl.: A61K 38/17, A61P 19/04

(54) **INHIBITORS OF TANGO1 ACTIVITY AND METHODS OF USE THEREOF**

(71) Applicant: Fundació Centre de Regulació Genòmica, 08003 Barcelona (ES); Universität zu Köln, 50923 Köln (DE); Fundació Institució Catalana De Recerca I Estudis Avançats, 08010 Barcelona (ES)
(72) Inventor: Malhotra, Vivek, Barcelona (US); Raote, Ishier, 08003 Barcelona (IN); Neundorf, Ines, 50923 Germany (DE); Krieg, Thomas, 50923 Germany (DE); Eckes, Beate, 50923 Germany (DE)
(74) Representative: Moore, Michael Richard

(57) **Abstract**

Uncontrolled secretion of extracellular matrix (ECM) proteins such as collagen can lead to excessive scarring and fibrosis and compromise tissue function. Despite the widespread occurrence of fibrotic diseases and scarring, effective therapies are lacking. The invention provides inhibitors of TANGO1 activity and methods of use thereof. In particular, the invention relates to peptide inhibitors capable of preventing TANGO1 heterodimerisation and/or homodimerisation and thereby prevent collagen export from the endoplasmic reticulum. The inhibitors of the invention are particularly useful for use in methods of treating fibrotic diseases, such as scleroderma.

## Description

### Field of the Invention

This invention relates to inhibitors of TANGO1 activity and methods of use thereof. In particular, the invention relates to peptide inhibitors capable of preventing TANGO1 heterodimerisation and/or homodimerisation and thereby prevent collagen export from the endoplasmic reticulum. The inhibitors of the invention are particularly useful for use in methods of treating fibrotic diseases, such as scleroderma.

### Background of the Invention

Uncontrolled secretion of extracellular matrix (ECM) proteins such as collagen can lead to excessive scarring and fibrosis and compromise tissue function. Despite the widespread occurrence of fibrotic diseases and scarring, effective therapies are lacking.

A major challenge in regenerative medicine is to optimize tissue regeneration by therapeutic modulation of its natural ability to form a scar after a mechanical injury or during disease processes. After tissue injury, platelets, endothelial cells and inflammatory cells induce fibroblasts to initiate extracellular matrix (ECM) deposition. Early granulation tissue is abundant in fibronectin and other ECM constituents. Fibrillar collagens form the main component of the final ECM and scar tissue.

Pathological scarring and fibrosis can develop in most tissues and represent a major cause of death, impaired quality of life, and economic burden, especially in aging societies. No therapy is as yet available and there is an urgent need to uncover new targets to treat scarring and fibrosis.

Scleroderma (systemic sclerosis), an autoimmune-driven, complex disease leading to extensive fibrosis of many organs including the skin is often used as a model disease to study principal mechanisms of fibrosis. Initial causes are still unknown but the disease reflects general features of fibrotic processes leading to activation of fibroblasts and excessive deposition of a collagen-rich ECM.

In spite of significant progress in understanding the pathophysiology of the underlying processes of fibrosis occurring in different organ systems and identifying the role of several fibrogenic cytokines, few specific therapeutic strategies are available to reduce or control connective tissue formation during wound healing and fibrosis. The presence of several concurrent fibrogenic signalling pathways may explain why trials of specific inhibitors of interleukin 4 (IL-4) or TGFβ pathways alone have yielded disappointing results as antifibrotic targets. However, all these fibrotic signal transduction cascades converge to produce at least one consistent hallmark of a broad range of pathologies - there is always excessive secretion of ECM proteins such as collagen. Modulation of this excess ECM deposition could therefore offer a promising disease-modifying, therapeutic approach.

Targeted control of collagen deposition requires a detailed understanding of the mechanisms governing the secretion of these large proteins. Of particular importance is their export from the endoplasmic reticulum (ER), a process that has only recently become amenable to molecular analysis after the discovery of the TANGO1 family of proteins, including TANGO1 and its paralog cTAGE5. TANGO1 is present in most metazoans and is required for collagen export from the ER in all animals tested thus far, including mammals (humans, canines, mice), Drosophila, and zebrafish.

Biologists have traditionally considered ER export un-druggable because its machinery is essential for cell viability and acts constitutively, such that targeting any component could produce pleiotropic, off-target effects on secretion. Inhibitors are herein disclosed which allow precise targeting of the interface of ERES proteins that controls their activity specifically, showing that it is possible to control ER export of specific ECM proteins, without major off-target toxicity.

The inhibitors of the present invention therefore provide a therapeutic intervention via targeted inhibition of ER export, for currently intractable fibrotic disorders characterized by excessive ECM production.

### Summary

The invention provides an inhibitor of TANGO1 activity, wherein the inhibitor is comprised of one or more inhibitors selected from the group consisting of a polypeptide, a small molecule, a nucleic acid, an antibody, and an antibody fragment.

The inhibitor of the invention may be an inhibitor of TANGO1 heterodimerisation and/or homodimerisation. In some embodiments, the inhibitor binds to one or more target proteins selected from the group consisting of TANGO1, TALI, and cTAGE5. In some embodiments, the inhibitor binds to a cytoplasmic portion of TANGO1, TALI, and/or cTAGE5, such as one or more cytoplasmic coiled-coil domains of the target protein. In some embodiments, the inhibitor binds to a region within SEQ ID NO: 17, SEQ ID NO: 18, SEQ ID NO: 22, and/or SEQ ID NO: 23.In some embodiments, the inhibitor comprises one or more polypeptides having at least 50%, at least 60%, at least 70%, at least 80%, at least 90%, at least 95%, at least 98%, or 100% sequence identity to the sequences selected from the group consisting of SEQ ID NO: 1 to SEQ ID NO: 10.

The inhibitor of the invention may be an inhibitor of TANGO1-mediated recruitment of ER-Golgi intermediate compartment (ERGIC) membranes. In some embodiments, the inhibitor binds to a NRZ tether protein selected from the group consisting of NBAS, RINT1, and ZW10. In some embodiments, the inhibitor binds to a region within SEQ ID NO: 27, SEQ ID NO: 28, or SEQ ID NO: 29. In some embodiments, the inhibitor comprises a polypeptide having at least 50%, at least 60%, at least 70%, at least 80%, at least 90%, at least 95%, at least 98%, or 100% sequence identity to SEQ ID NO: 1 or SEQ ID NO: 2.

The inhibitor of the invention may comprise one or more signal sequences. In some embodiments, the one or more signal sequences comprise a polypeptide selected from the group consisting of SEQ ID NO: 13, SEQ ID NO: 30, SEQ ID NO: 31, SEQ ID NO: 32, SEQ ID NO: 33, SEQ ID NO: 34, SEQ ID NO: 35, and SEQ ID NO: 36.

In some embodiments where the inhibitor of the invention comprises RNA, the RNA is selected from the group consisting of shRNA, siRNA, miRNA, antisense oligonucleotides, RNA aptamers, modified RNA, native RNA, and combinations thereof.

In some embodiments, the inhibitor of the invention may be a combination of at least two inhibitors described herein
The invention further provides vectors encoding the inhibitor(s) described herein. In some embodiments, the vector may be selected from the group consisting of plasmid, adenovirus, adeno-associated virus (AAV), lentivrus (LV), cytomegalovirus (CMV), herpes simplex virus (HSV), vesicular stomatitis virus (VSV), and baculovirus. The invention further provides expression cassettes encoding the inhibitor(s) described herein. The invention further provides mRNA encoding the inhibitor(s) described herein. In some embodiments, the mRNA may be comprised within an RNA vaccine, a circular RNA, an RNA nanostructure, or an RNA/DNA nanostructure.

The invention further provides delivery vehicles comprising the inhibitor(s), vectors, expression cassettes, and/or mRNA described herein. In some embodiments, the delivery vehicle may be selected from the group consisting of a nanoparticle, a vesicle, a nanovesicle, a lipid nanoparticle, a lipid nanovesicle, a liposome, and an exosome. The invention also provides pharmaceutical compositions comprising inhibitor(s), vectors, expression cassettes, mRNA, and/or delivery vehicles described herein, further comprising a pharmaceutically acceptable excipient.

The invention provides therapeutic agents comprising inhibitor(s), vectors, expression cassettes, mRNA, delivery vehicles, and/or pharmaceutical compositions described herein, for use in medicine. In some embodiments, the therapeutic agents may be for use in a method of treating a fibrotic disease or disorder in a subject, the method comprising administering the therapeutic agent to a subject in need thereof. The fibrotic disease or disorder may be one or more selected from the group consisting of a scarring, sclerosing and fibrosing disease or disorder, optionally wherein the disease or disorder is selected from the group consisting of scleroderma, local scleroderma, dermatofibrosis lenticularis disseminata, morphea, keloids, peyronie's disease, nephrogenic systemic fibrosis, scarring, idiopathic pulmonary fibrosis, non-idiopathic pulmonary fibrosis, chronic kidney disease, systemic scleroderma skin fibrosis, hypertrophic scars, liver fibrosis, and cardiovascular disease.

The invention further provides a method of screening for an inhibitor of TANGO1 activity, the method comprising (a) providing one or more target regions for inhibition of TANGO1 activity, wherein the target regions are selected from a group comprising SEQ ID NOs: 14 to 29; (b) providing a library of candidate inhibitors, optionally wherein the candidate inhibitors comprise candidate inhibitors selected from the group consisting of SEQ ID NOs: 1 to 10; (c) contacting the candidate inhibitors with the one or more target regions; and (d) assessing the ability of candidate inhibitors to bind to the one or more target regions.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible.

### Brief Description of the Drawings

The invention is further illustrated by way of the accompanying drawings, described below.

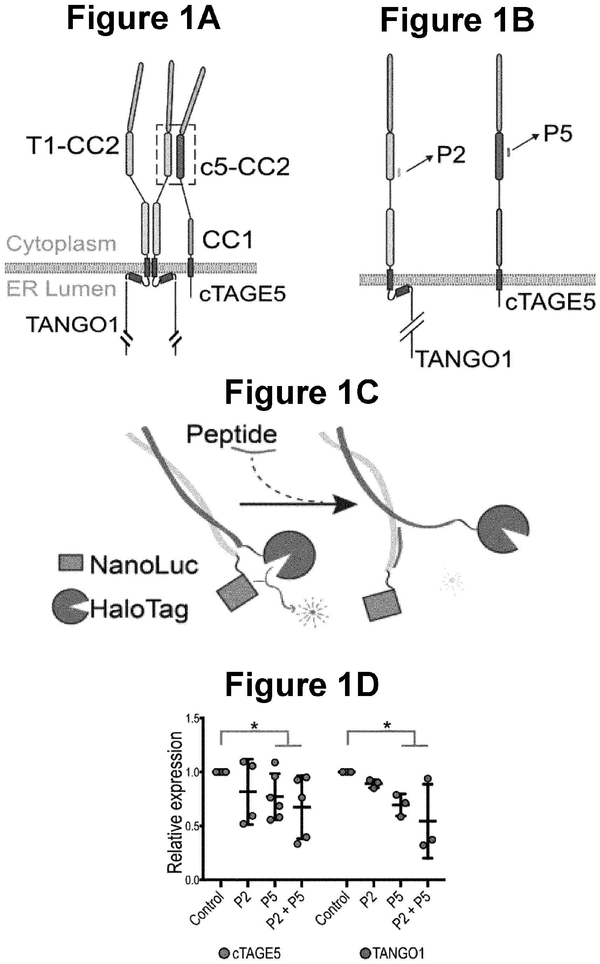

**Figure 1** relates to the design of peptides to inhibit TANGO1 heterodimerisation. **Fig. 1A** depicts the organization and the domains of TANGO1 and cTAGE5. Each of these proteins contain two cytoplasmic coiled-coil domains. TANGO1 coiled-coil 2 (T1-CC2) is highlighted in green (far left). cTAGE5 CC2 in magenta (far right). **Fig. 1B** is a schematic indicating where the inhibitor peptide sequences map to in TANGO1 and cTAGE5. **Fig. 1C** depicts a schematic of the NanoBRET assay to test whether peptides inhibit interactions between the TANGO1-cTAGE5 CC2 **Fig. 1D** shows quantification of Western blots of U2OS cell lysates treated with P2, P5 or P2+P5, probed for cTAGE5, and U2OS cell lysates treated with P2, P5 or P2+P5, probed for TANGO1. β-tubulin was used as a loading control. Graph shows TANGO1 (green/right hand columns of each pair) and cTAGE5 (magenta/left hand columns of each pair) levels (mean +/- SD) in control or peptide-treated cells (normalized to control) N=3.

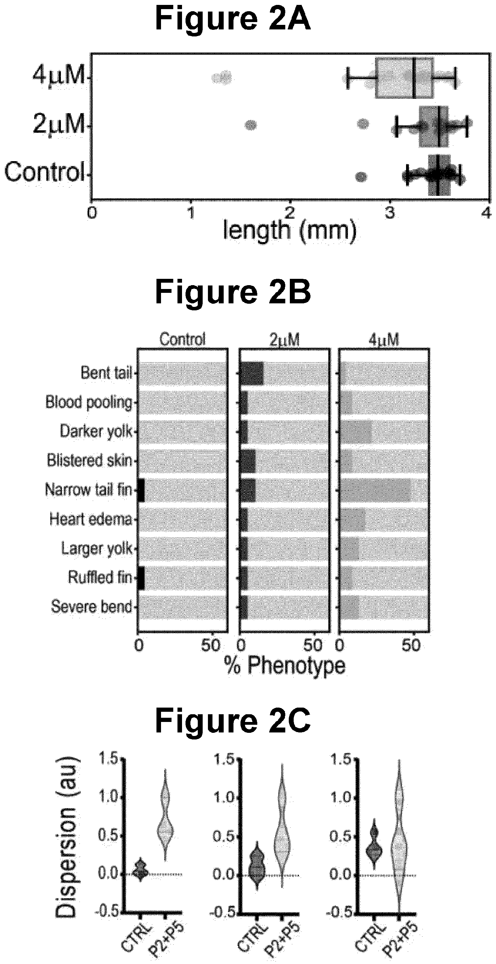

**Figure 2** shows phenotypic effects of peptide treatment on zebrafish larvae. **Fig. 2A** shows quantification of zebrafish larva length from head to tail (boxplot with median and interquartile range). Zebrafish larvae at 3 dpf. Fish were treated with P2 + P5 (4µM), or left untreated in E3 (control). **Fig. 2B** shows quantification of phenotypes observed in fish for n = 21, 19, 23 replicates in control, 2µM and 4µM respectively, from three independent experiments. **Fig 2C** shows quantification of collagen fiber direction obtained from the SHG signal in the three segmented areas. Collagen fiber dispersion distribution plots from a representative control or treated fish. This measure represents the dispersion of the preferred orientation of collagen fibers imaged as described in Example 2. Control measurements are represented in black and P2+5 (4µM)-treated in orange. Kolmogorov-Smirnov test *** p<0.001.

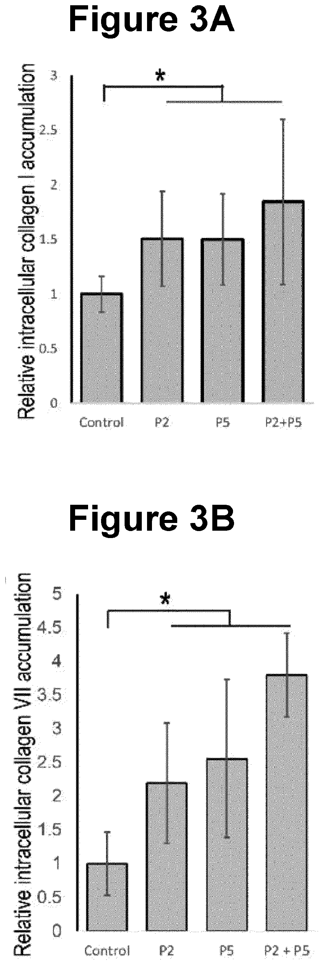

**Figure 3A** shows quantification of intracellular collagen I accumulation (normalized to control) in U2OS cells treated with peptides P2 and P5. *p<0.05, Students' t-test. **Fig. 3B** shows quantification of intracellular collagen VII accumulation (normalized to control) in RDEB/FB/C7 cells treated with peptides P2 and P5. *p<0.05, Students' t-test.

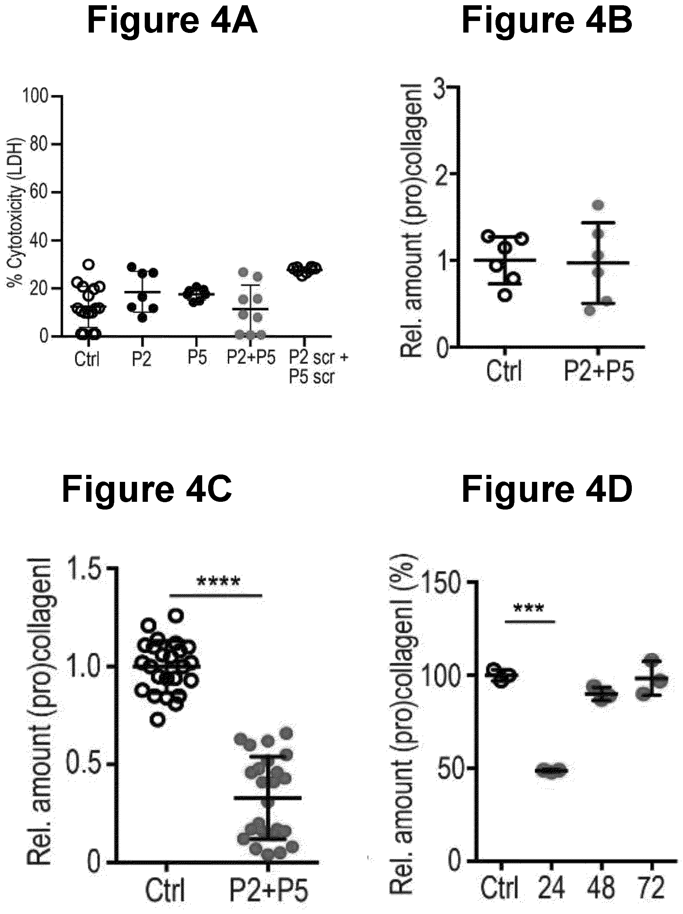

**Figure 4A** shows the results of an LDH release cytotoxicity assay. Primary cells incubated with the peptides did not exhibit toxic effects. N = 6. **Fig. 4B** shows the quantification of Western blotting of fibroblast lysates treated or not (Ctrl) with P2+P5 at 40 µM for 20 h. N = 6 independent experiments. **Fig. 4C** shows the quantification of Western blotting and quantification of untreated and P2+P5 treated skin fibroblasts. N >20 independent experiments; Mann Whitney U test, p<0.0001. **Fig 4D** depicts the duration of inhibitory effect after removal of P2+P5. Levels of untreated (Ctrl) were set at 100%. N = 3 per time point, 2-way ANOVA with Sidak's multiple comparisons test. *** p<0.001.

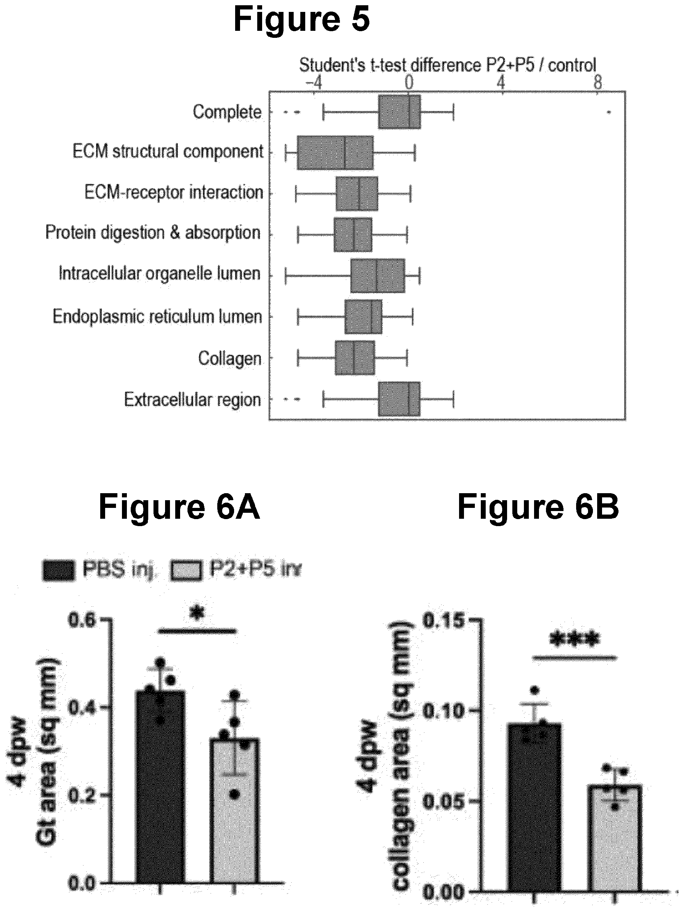

**Figure 5** shows Gene ontology (GO) terms of proteins affected by P2+P5 peptide treatment.
**Figure 6** shows results of zebrafish wound sites at 4 dpw treated with PBS (control) and P2+P5 Granulation tissue (gt) area was measured. N=5 fish per treatment. **Fig. 6A** shows quantification of granulation tissue area in control or peptide-treated animals. * p < 0.05, student's t-test. **Fig. 6B** shows quantification of collagen area in the granulation tissue. *** p < 0.001.

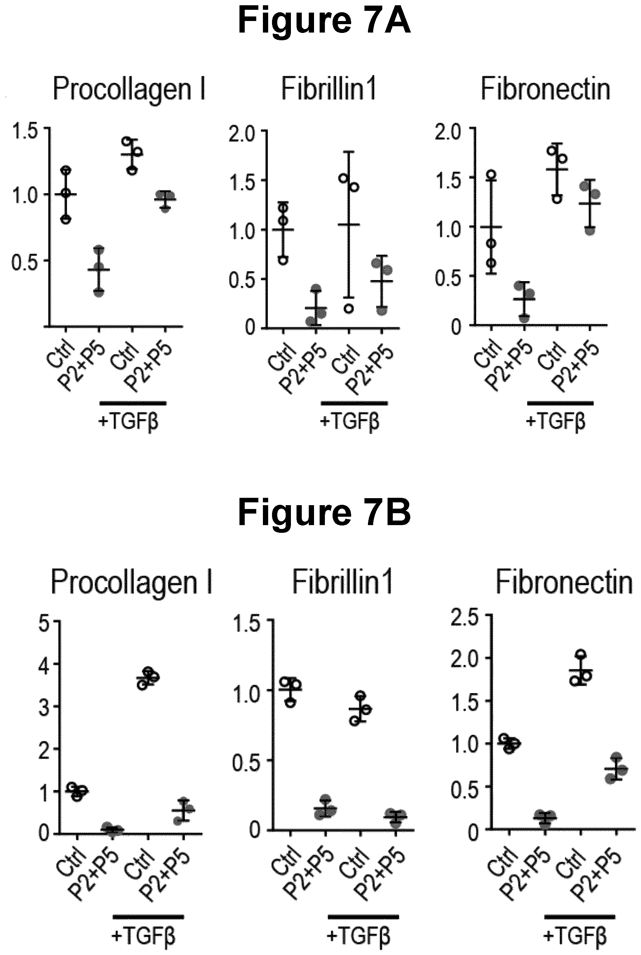

**Figure 7** depicts the quantification of Western blots of supernatants from fibroblasts incubated for 20 h in the absence of serum (Ctrl) or with 40 µM each of P2+P5 and without or with ng/ml TGFβ. Peptide treatment strongly reduces amounts of secreted procollagen I, fibrillin1 and fibronectin in fibroblasts from healthy donors (shown in **Fig. 7A**) and scleroderma patients (shown in **Fig. 7B**) before and particularly after stimulation of ECM production by TGFβ.

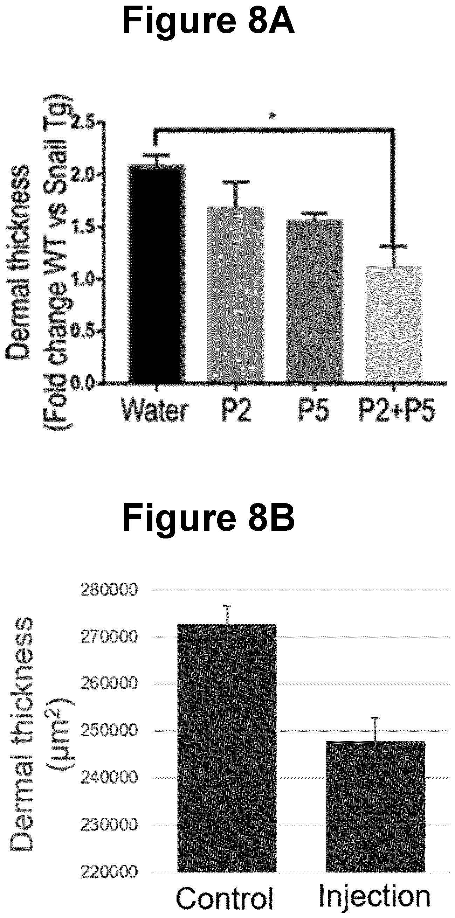

**Figure 8** presents results showing that the inhibitors prevent skin thickening in a mouse model of skin fibrosis as described in Example 8. **Fig. 8A** shows dermal thickness of Snail transgenic skin injected with water, P2, P5 and P2+P5, as a proportion of wild type counterpart dermal thickness. Quantification of dermal thickness as a fold change, WT to Snail Tg. * p < 0.05 student's t test. **Fig. 8B** shows dermal thickness in µm of Snail transgenic skin injected with P1 inhibitor (SEQ ID NO: 2) and control (SEQ ID NO: 13).

### Detailed Description of the Invention

All references cited herein are incorporated by reference in their entirety. Unless otherwise defined, all technical and scientific terms used herein have the same meaning as commonly understood by one of ordinary skill in the art to which this invention belongs.

Unless otherwise indicated, the practice of the present invention employs conventional techniques in chemistry and chemical methods, biochemistry, pharmaceutical formulation, and delivery and treatment regimes for patients, which are within the capabilities of a person of ordinary skill in the art. Such techniques are also described in the literature cited herein, each of which is herein incorporated by reference.

Prior to setting forth the detailed description of the invention, a number of definitions are provided that will assist in the understanding of the invention.

As used herein, the term 'inhibitor' refers to an agent that is capable of slowing down, impeding, or preventing a specific biological, chemical, or enzymatic process. The inhibitors disclosed herein may act through various mechanisms, including but not limited to competitive, non-competitive, or uncompetitive binding to target molecules, interference with catalytic sites, steric disruption, or modulation of regulatory pathways. The use of the term 'inhibitor' encompasses compounds that exhibit inhibitory effects on the intended target, thereby providing a means for controlling, modulating, or influencing the activity or function associated with said target. Furthermore, the term 'inhibitor' as used in this application is not limited to any particular class of chemical compounds and includes, but is not limited to, small molecules, peptides, nucleic acids, antibodies, antibody fragments. An inhibitor of TANGO1 heterodimerisation, as used herein, may refer to an inhibitor which prevents a TANGO1 heterodimer forming, an inhibitor which allows a TANGO1 heterodimer to form albeit in a non-functional manner, or an inhibitor which targets an extant TANGO1 heterodimer to inhibit its function.

As used herein, the term 'activity' in relation to a protein and/or gene refers to any process, action, interaction, reaction, catalysis, or inhibition in which the protein, gene, or gene product is involved or associated with. For instance, the term 'TANGO1 activity' includes all processes and pathways in which TANGO1 is implicated, including TANGO1 homodimerisation, TANGO1 heterodimerisation (for instance with cTAGE5 and/or TALI), TANGO1-mediated recruitment of ER-Golgi intermediate compartment (ERGIC) membranes, TANGO1 binding of and interaction with NBAS, TANGO1 binding of and interaction with RINT1, and TANGO1 binding of and interaction with ZW10.

As used herein, terms 'bind', 'target', and/or 'interact' may be used interchangeably.

An 'antibody' denotes a protein that is produced in response to an antigen that is able to combine with and bind to the antigen, preferably at a specific site on the antigen, known as an epitope. The term as used herein includes antibodies of polyclonal and monoclonal origin, unless stated otherwise, as well as antibody equivalent technologies. Polyclonal antibodies are a group of antibodies produced by different B lymphocytes in response to the same antigen; different antibodies in the group typically recognize different parts (epitopes) on the antigen. A monoclonal antibody recognizes only one type of antigen and is produced by the daughter cells of a single antibody-producing lymphocyte, typically a hybridoma. Also included within the term 'antibody' are antigen binding fragments of naturally or non-naturally occurring antibodies, for example, the `Fab fragment', 'Fab' fragment' (a Fab with a heavy chain hinge region) and 'F(ab')2 fragment' (a dimer of Fab' fragments joined by a heavy chain hinge region). Recombinant methods have been used to generate small antigen-binding fragments, such as 'single chain Fv' (variable fragment) or 'scFv,' consisting of a variable region light chain and variable region heavy chain joined by a synthetic peptide linker. Unlike antibodies derived from other mammals, camelid species express fully functional, highly specific antibodies that are devoid of light chain sequences. Camelid heavy chain antibodies are of particular use, as they are found as homodimers of a single heavy chain, dimerized via their constant regions. The variable domains of camelid heavy chain antibodies are referred to as VHH domains and retain the ability, when isolated as small fragments of the VH chain, to bind antigen with high specificity (Hamers-Casterman et al., 1993, Nature 363: 446-448; Gahroudi et al., 1997, FEBS Lett. 414: 521-526). Further included within the term 'antibody' are so called camelized mutants of human VH domains that retain antigen binding activity but exhibit some of the advantages of camelid VHH domains (Riechmann, 1994, FEBS Lett. 339: 285-290). In addition to antigen binding fragments, antibodies of the present invention can include derivatives of antibodies, such as chimeric fusions with labelling moieties including green fluorescent protein (GFP).

As used herein, the term 'nucleic acid' refers to a biopolymer composed of nucleotide units, each consisting of a sugar molecule, a phosphate group, and a nitrogenous base. Nucleic acids include, but are not limited to, deoxyribonucleic acid (DNA), ribonucleic acid (RNA), and xeno nucleic acids (XNA). The term encompasses naturally occurring sequences as well as synthetically engineered or modified nucleic acid molecules, including fragments, derivatives, analogues, and combinations thereof.

As used herein, the term 'TANGO1' refers to one or both of the transport and Golgi organization protein 1 (TANGO1) encoded by the gene MIA3, or a fragment thereof. The term therefore encompasses both the short and long isoforms of the protein having sequences according to SEQ ID NO: 14 and SEQ ID NO: 20, respectively. In other words, unless otherwise specified, TANGO1 may be taken to mean TANGO1 long and/or TANGO1 short. The term 'TANGO1', as used herein, further encompasses proteins having high sequence identity to SEQ ID NO: 14 and/or SEQ ID NO: 20, which are functionally identical to TANGO1. High sequence identity includes proteins having at least 80%, at least 85%, at least 90%, at least 95%, at least 98%, or at least 99% identity to SEQ ID NO: 14 and/or SEQ ID NO: 20. Thus as used herein the term 'TANGO1' includes proteins exhibiting minor mutations with relation to SEQ ID NO: 14 and/or SEQ ID NO: 20. TANGO1 is synonymous with C219-reactive peptide1, D3201, and Melanoma inhibitory activity protein 3. MIA3 is synonymous with KIAA0268, TANGO, and UNQ6077/PRO20088.

As used herein, the term `cTAGE5' refers to the cutaneous T-cell lymphoma-associated antigen 5 encoded by the MIA2 gene, or a fragment thereof. `cTAGE5' may have a sequence according to SEQ ID NO: 21, however the term `cTAGE5' as used herein, further encompasses proteins having high sequence identity to SEQ ID NO: 21, which are functionally identical to cTAGE5. High sequence identity includes proteins having at least 80%, at least 85%, at least 90%, at least 95%, at least 98%, or at least 99% identity to SEQ ID NO: 21. cTAGE5 is synonymous with Melanoma inhibitory activity protein 2; MIA protein 2; CTAGE family member 5 ER export factor, Cutaneous T-cell lymphoma-associated antigen 5, and Meningioma-expressed antigen 6/11. MIA2 is synonymous with CTAGE5, MEA11, MEA6, MGEA11, and MGEA6.

As used herein the term 'TALI' refers to the TANGO1-like protein encoded by the MIA2 gene, or a fragment thereof. 'TALI' also refers to a chimeric protein resulting from the fusion of MIA2 and cTAGE5 gene products. 'TALI' may have a sequence according to SEQ ID NO: 25, however the term 'TALI' as used herein, further encompasses proteins having high sequence identity to SEQ ID NO: 25, which are functionally identical to TALI. High sequence identity includes proteins having at least 80%, at least 85%, at least 90%, at least 95%, at least 98%, or at least 99% identity to SEQ ID NO: 25. TALI and cTAGE5 are both encoded by MIA2. TALI is also identified by uniprot isoform 1 Q96PC5-3.

As used herein, the term 'heterodimerisation' refers to any interaction between at least two different subunits. The subunits may be proteins and/or nucleic acids. The term 'heterodimerisation' and 'bind' (or any other grammatical conjugation thereof), encompass interactions in the form of a covalent bond, an ionic bond, a hydrogen bond, any kind of electrostatic interaction or bond, any kind of steric interaction, any kind of interaction which initiates conformational change in one or both subunits, any kind of interaction which results in a tertiary or quaternary structure being formed by the subunits, coiled-coil interactions through alpha-helices, helix-loop-helix (HLH) domain interaction, leucine zipper motif-mediated dimerisation, β-barrel domain interaction, disulfide bond formation, domain-swapping, phosphorylation-mediated dimerisation, hydrophobic interactions, metal ion coordination, or oligomerisation domains.

As used herein, the term 'sequence identity' refers to the percentage similarity between two nucleotide or amino acid sequences, calculated by aligning the sequences and identifying matching residues. Sequence identity provides a measure of the degree of similarity or identity between the sequences, with a higher percentage indicating greater similarity. The alignment may consider substitutions, insertions, or deletions, and the resulting identity value is expressed as a percentage of identical residues relative to the total length of the shorter sequence. The sequence identity may further take into account similar properties between different amino acids or residues, such as hydrophobicity, chain length, functional groups, impact on solubility, etc. This definition encompasses various applications, including but not limited to the comparison of genetic sequences, the evaluation of homologous proteins, and the assessment of the similarity between nucleic acid molecules. Sequence identity can also be to a fragment or portion of the full length polynucleotide or polypeptide. Hence, a sequence may have only 50% overall sequence identity with a sequence of the invention but in a particular region, domain or subunit could share 80%, 90%, or as much as 99% sequence identity with sequence of the invention. It is understood that sequence identity may be determined using standard bioinformatics tools and algorithms, and the specific parameters for sequence alignment will be disclosed in accordance with the technical context of the invention disclosed in this application.

The term 'antisense oligonucleotide' (AON) refers to a short oligonucleotide or modified oligonucleotide which is antisense to and binds to (i.e. hybridises to) a target region of a polynucleotide, such as a gene transcript, pre-mRNA, mRNA or RNA fragment. According to the invention, the target region for an AON is preferably in a pre-mRNA, i.e. the AON binds to a target region on a transcript before the splicing process takes place. The AONs of the invention may comprise native or modified RNA, DNA, or mixtures thereof. Any modification may be naturally occurring or non-naturally occurring. Any references to an AON sequence provided as an RNA sequence is intended to also encompass the equivalent DNA sequence. In particular, any sequence comprising U bases is intended to refer equally to the corresponding sequence in which T bases are present in place of one or more (e.g. all) of the Us. In embodiments, the oligonucleotides according to the invention may comprise nucleotides comprising inosine. In particular, any AON sequence disclosed herein is intended to encompass nucleotide sequences where one or more of the T, G or As are replaced with I, as far as this is compatible with the function of the AON. The AONs according to the invention have an effect on the regulation of alternative splicing. In particular, the AONs according to the invention advantageously promote the skipping of a specific exon, resulting in a decreased inclusion of the exon in the mature transcript resulting from splicing of the pre-mRNA. As the skilled person would understand, the range of lengths of AONs that are suitable will depend on the desired specificity (where longer AONs are expected to bind to their target sequence with higher specificity) and efficacy of the *in vivo* delivery (where longer AONs are expected to be more difficult to efficiently deliver to the target cells). The AON's of the invention preferably have a length of at most 35 nucleotides, at most 25 nucleotides, at most 24 nucleotides, at most 21 nucleotides, or at most 18 nucleotides. An oligonucleotide as used herein may have a length of at least 7 nucleotides, at least 10 nucleotides, at least 13 nucleotides, at least 14 nucleotides, or at least 17 nucleotides.

As known in the art, the AONs of the invention may be chemically modified, for example to increase their stability, reduce their immunogenicity, increase their binding affinity to a target sequence, reduce their non-specific binding to unintended targets (see e.g. Mou & Gray, 2002) and/or enhance their delivery, etc. Chemical modification of an oligonucleotide refers to a chemical difference compared to the native form of ribo or deoxyribonucelotides (i.e. nucleotides comprising the naturally occurring nucleobases of RNA or DNA, including (deoxy)adenosine, (deoxy)guanosine, (deoxy)thymidine, (deoxy)cytidine, 5-methyl (deoxy)cytidine and uridine; and a phosphate linking group). Chemical modifications may be applied to the sugar moiety of a nucleoside, the nucleobase moiety of a nucleoside and/or the phosphate backbone. Examples of modified chemically modified nucleotides or analogues that may be used in the present invention include locked nucleic acids, the use of a phosphorothioate backbone, 2'-O-methylated nucleotides, 2'-O-methoxyethyl modified (2'MOE) nucleotides, methylated cytosine, constrained ethyl (cET) nucleic acids, bridged nucleic acids (BNAs), phosphorodiamidate morpholino oligomers (PMOs), peptide nucleic aids (PNAs), cyclohexene nucleic acids (CeNAs), tricycle-DNA (tcDNA), N3'-P5' phosphoroamidates (NPs), 2'-fluoro-2'-deoxyadenosine-5'-triphosphate, 2'-fluoro-2'-deoxycytidine-5'-triphosphate, 2'-fluoro-2'-deoxyguanosine-5'-triphosphate, 2'-fluoro-2'-deoxyuridine-5'-triphosphate, 2'-fluoro-2'-deoxythymidine-5'-triphosphate, 2'-O-methyladenosine-5'-triphosphate, 2'-O-methylcytidine-5'-triphosphate, 2'-O-methylguanosine-5'-triphosphate, 2'-O-methyluridine-5'-triphosphate , 2'-O-methylinosine-5'-triphosphate, 2'-O-methyl-2-aminoadenosine-5'-triphosphate, 2'-O-methylpseudouridine-5'-triphosphate, 2'-O-methyl-5-methyluridine-5'-triphosphate etc. Chemical modifications may be applied to one or more nucleotides of an oligonucleotide and, as such, reference to a 'modified (antisense) oligonucleotide' or 'modified AON' relates to oligonucleotides comprising at least one modified nucleotide. In embodiments, 2'-O modified nucleotides may be used to surround (i.e. flank) a central sequence, thereby forming a gapmer, as known in the art. Such compounds may be particularly resistant to nuclease degradation. As the skilled person would understand, multiple modifications may be present on the same nucleotide. Further, different modifications may be present on different nucleotides of the same oligonucleotide. As known in the art, the optimal length of an AON may depend on the modifications (or absence thereof) that may be present on the AON.

A 'locked nucleic acid' (LNA) as used herein refers to a ribonucleic acid where at least one of the nucleotides has the ribose moiety modified with a methylene bridge connecting the 2' oxygen and the 4' carbon. Without wishing to be bound by theory, it is believed that this locks the ribose ring in an ideal conformation for Watson-Crick base-pairing, making the pairing of a locked nucleotide with a complementary nucleotide strand more rapid and more stable. A locked nucleic acid may comprise a mixture of locked nucleotides and ribonucleic acids. In embodiments, all of the nucleotides of the oligonucleotides according to the invention are locked nucleotides. In embodiments where the AON is an LNA, the length of the AON may be between 7 and 21 nucleotides. In some embodiments, the length of the AON may be between 7 and 17 nucleotides. In some such embodiments, the length of the AON is 13 or 14 nucleotides.

As used herein the term 'region', may refer to one or more residues, amino acids, or nucleotides in a sequence. As such, a 'region' may be a single amino acid residue in a protein.

As used herein the term 'appended', may refer to the inclusion of a sequence (such as a signal sequence) in a polypeptide or polynucleotide sequence. For example, the appended sequence is included in such a way that the two sequences are joined as a whole, individual sequence. As such, the term 'appended' may refer to the appended sequence being bound directly at the C' or N' terminus of the other sequence. Alternatively, 'appended' may include the appended sequence being bound to an intermediate sequence disposed between the appended sequence and the other sequence. The intermediate sequence may be any length or form. The term 'appended' also refers to a sequence (such as a signal sequence) which is associated with another sequence without necessarily being bound to the sequence to which it is appended. For example, the appended sequence may be attached to the other sequence by a linker peptide, molecule, or sequence; the appended sequence may be associated with the other sequence within a carrier molecule such as a vesicle, nanovesicle, nanoparticle, exosome, liposome, or lipid; the appended sequence may be on the exterior of a carrier molecule containing the other sequence; or the appended sequence may be present on a larger molecule which also comprises the other sequence, for instance a protein molecule containing both the appended sequence and the other sequence, or a DNA, RNA, or RNA/DNA nanostructure which comprises both the appended sequence and the other sequence.

As used herein, the term 'recruit' or 'recruitment' may refer to any interaction between proteins which results in activation, translocation, or conformational change of one or more proteins involved in the interaction. The term 'recruitment' therefore encompasses catalytic activity of any kind, especially that which permits a cascade of downstream events or reactions.

As used herein, the term 'ER-Golgi intermediate compartment (ERGIC) membranes' or vesicular-tubular cluster (VTC) refers to a compartment or organelle in eukaryotic cells, comprising a cluster of vesicular and tubular structures that facilitates trafficking and sorting of cargoes at the interface of the endoplasmic reticulum (ER) and Golgi complex.

As used herein, the term `vector' refers to any vehicle which may encode a sequence for transcription and/or translation within a cell. For example, the vector may encode all or part of the sequence of an inhibitor according to the present invention. The term `vector' therefore encompasses plasmid vectors, bacterial artificial chromosomes (BACs), yeast artificial chromosomes (YACs), viral vectors (e.g., adenovirus, adeno-associated virus), lentiviral vectors, retroviral vectors, expression vectors, cytomegaloviral vectors, herpes simplex viral vectors, Sendai viral vectors, vesicular stomatitis viral vectors, baculoviral vectors, transposons, cosmid vectors, phage vectors (e.g., lambda phage), mRNA, synthetic mRNA, circular RNA, RNA nanostructures, DNA nanostructures, and RNA/DNA nanostructures.

The term 'expression cassette' is used to denote a DNA or RNA molecule that is either linear or circular, into which another DNA or RNA sequence fragment of appropriate size can be integrated. Such DNA fragment(s) can include additional segments that provide for transcription of a gene encoded by the DNA sequence fragment. The additional segments can include and are not limited to: promoters, transcription terminators, enhancers, internal ribosome entry sites, untranslated regions, polyadenylation signals, selectable markers, origins of replication and such like. Expression vectors are often derived from plasmids, cosmids, viral vectors and yeast artificial chromosomes; vectors are often recombinant molecules containing DNA sequences from several sources.

A 'polynucleotide' is a single or double stranded covalently-linked sequence of nucleotides in which the 3' and 5' ends on each nucleotide are joined by phosphodiester bonds. The polynucleotide may be made up of deoxyribonucleotide bases or ribonucleotide bases. Polynucleotides include DNA and RNA, and may be manufactured synthetically in vitro or isolated from natural sources. Sizes of polynucleotides are typically expressed as the number of base pairs (bp) for double stranded polynucleotides, or in the case of single stranded polynucleotides as the number of nucleotides (nt). One thousand bp or nt equal a kilobase (kb). Polynucleotides of less than around 40 nucleotides in length are typically called 'oligonucleotides'.

A 'polypeptide' is a polymer of amino acid residues joined by peptide bonds, whether produced naturally or by synthetic means. As used herein, the term 'polypeptide' is interchangeable with the term 'peptide'. The term 'polypeptide' as used herein denotes the product of a naturally occurring polypeptide, precursor form or proprotein. Polypeptides also undergo maturation or post-translational modification processes that may include, but are not limited to: glycosylation, proteolytic cleavage, lipidization, signal peptide cleavage, propeptide cleavage, phosphorylation, and such like. A 'protein' is a macromolecule comprising one or more polypeptide chains.

The term `promoter' as used herein denotes a site on DNA to which RNA polymerase will bind and initiate transcription. Promoters are commonly, but not always, located in the 5' non-coding regions of genes.

The terms 'treat' or 'treating,' as used herein, refers to partially or completely alleviating, inhibiting, ameliorating and/or relieving a disorder or condition, or one or more symptoms of the disorder or condition. As used herein, the terms 'treatment,' 'treat,' and 'treating' refer to partially or completely alleviating, inhibiting, ameliorating and/or relieving a disorder or condition, or one or more symptoms of the disorder or condition, as described herein. In some embodiments, treatment may be administered after one or more symptoms have developed. In some embodiments, the term 'treating' includes halting the progression of a disease or disorder. Treatment may also be continued after symptoms have resolved, for example to prevent or delay their recurrence. Thus, in some embodiments, the term 'treating' includes preventing relapse or recurrence of a disease or disorder.

As used herein, the term 'pharmaceutically acceptable excipient' refers to refers to a substance that is included in a pharmaceutical formulation to serve as a carrier, vehicle, or stabiliser for the active ingredient (drug or therapeutic agent). Excipients may be chosen based on their safety, compatibility, and inert nature with the active substance, such that they do not interfere with the therapeutic action of the drug. Pharmaceutically acceptable excipients may include a variety of substances such as fillers, binders, diluents, disintegrants, lubricants, and stabilizers.

Pharmaceutical preparations of the invention are formulated to conform with regulatory standards and can be administered orally, intra-venously, topically, or via other standard routes. The pharmaceutical preparations may be in the form of tablets, pills, edible films, lotions, gels, liquids, powders, suppositories, suspensions, liposomes, microparticles or other suitable formulations known in the art.

As used herein, the term 'topical administration' refers to the administration of a therapeutic directly to the body, for example directly onto the skin. Formulations for topical administration include, but are not limited to, creams, lotions, patches, ointments, balms, foams, sprays, and gels.

As used herein, the term 'wound' is broadly defined as any injury, damage, or disruption to the integrity of the body's tissues, encompassing a wide array of conditions across various anatomical locations. Wounds may result from physical trauma, surgical procedures, pathological processes, or environmental factors, and can manifest in diverse forms and severity levels, including but not limited to incisions, abrasions, lacerations, punctures, contusions, avulsions, burns (e.g. thermal, chemical, or electrical), ulcers (including pressure ulcers), surgical wounds (e.g. post-operative incisions), gunshot wounds, bites (e.g. animal or insect), crush injuries, penetrating injuries, fractures with open skin wounds, and chronic wounds (e.g. diabetic ulcers, venous ulcers, arterial ulcers).

As used herein, the term 'scarring' or `scar' refers to any aggregation or collection of fibrous tissue(s) or fibrous protein(s) (such as collagens, keratins, myosins, elastins, and other ECM proteins). Reducing scarring therefore encompasses a reduction in the number or concentration of fibrous tissue(s) and/or fibrous protein(s). The term 'scarring' or 'scar' also refers to the presentation of a healed wound or trauma site which has been subject to increased levels of fibrous tissue(s) and/or fibrous protein(s), for example as a visible scar on the skin of an individual. Further, 'scar' or 'scarring' may refer to a fibrotic reaction associated with and/or clinical presentation of autoimmune disease, inflammatory reaction (such as chronic inflammatory reaction), fibrosing diseases and disorders, and/or sclerosing diseases and disorders. The terms 'scar' or 'scarring' as used herein therefore encompass fibrosis and/or sclerosis.

As used herein, the term 'skin blemish' refers to an imperfection, discoloration, or irregularity on the surface of the skin. Therefore, the term 'skin blemish' encompass a variety of conditions, including but not limited to acne lesions, scars, hyperpigmentation, wrinkles, and other noticeable deviations from the skin's natural appearance. These imperfections can arise from various factors such as inflammation, hormonal changes, sun exposure, or injury.

As used herein, the term 'comprising' means any of the recited elements are necessarily included and other elements may optionally be included as well. 'Consisting essentially of' means any recited elements are necessarily included, elements that would materially affect the basic and novel characteristics of the listed elements are excluded, and other elements may optionally be included. 'Consisting of' means that all elements other than those listed are excluded. Embodiments defined by each of these terms are within the scope of this invention.

As used herein, all references to elements, components, or features in the singular form are intended to encompass their corresponding plural forms and vice versa. For example, the term 'inhibitor' should be understood to include both individual inhibitors and multiple instances of such inhibitors. The singular and plural forms are interchangeable throughout this disclosure, and any variations should be considered within the scope of the claims appended hereto.

The present invention is based in part upon the discovery that inhibitors that target and disrupt the heterodimerisation of TANGO1 may be used to modulate the export of ECM components from endoplasmic reticulum exit sites (ERES). The present invention is further based in part upon the discovery that inhibitors that target TANGO1 may disrupt TANGO1-mediated recruitment of ER-Golgi intermediate compartment (ERGIC) membranes. The present invention therefore provides inhibitors capable of therapeutic modulation of ERES function in ECM hypersecretion.

By inhibiting the function of the master regulators of the collagen endoplasmic (ER)-exit machinery, i.e. the transport and Golgi organization protein 1 (TANGO1) family of proteins comprising TANGO1, TANGO1-short, cutaneous T-cell lymphoma-associated antigen 5 (cTAGE5), TANGO1-like protein (TALI) (TANGO1/TANGO1-short and cTAGE are expressed in all tissues; TALI is expressed only in lung, testis, intestine, colon, pancreas, kidney, liver and prostate), the invention also provides a potential therapeutic approach to control fibrosis by targeting collagen hypersecretion.

TANGO1 is a transmembrane protein localized to the ER exit site. It binds collagens in the ER lumen via its SRC Homology 3 like domain via heat shock protein (HSP) in vertebrates. In the cytoplasm, its proline rich domain (PRD) binds to the inner layer of COPII coat proteins Sec23 and Sec16 that recruits TANGO1 to the ER exit site. Alternative splicing of TANGO1 results in a short isoform, TANGO1-short, lacking the lumenal domain. On the cytoplasmic side of the ER membrane, TANGO1/ TANGO1-short have two coiled-coil (CC) domains (CC1 and CC2). CC1 is used by TANGO1/TANGO1-short to recruit ER-Golgi intermediate compartment (ERGIC) membranes by interacting with the NBAS, RINT1, ZW10 (NRZ) protein tether complex for producing a collagen carrier. TANGO1 CC1 also mediates homodimerization of TANGO1. TANGO1/TANGO1-short CC2 binds to a similar CC2-like domain in the closely related cTAGE5. Like the TANGO1/TANGO1-short PRDs, the cTAGE5 PRD also interacts with Sec23. Thus, TANGO1/TANGO1-short and cTAGE5 together function to create the exit route for bulky collagens.

Secreted collagens compose 15-20% of dry protein weight in mammals, and are required for formation of extracellular matrix and basement membrane. There are 28 different kinds of collagens that are secreted and defects in collagen secretion or mutation are therefore the main pathophysiological hallmark of a broad range of diseases encompassing collagenopathies (connective tissue disorders, in which mutations in collagen genes hamper its proper synthesis)) and fibrosis. Fibrosis consists of excessive and uncontrolled scar tissue formation through accumulation of (myo)fibroblasts and dysregulated deposition of extracellular matrix-with secreted collagens being the most abundant component. This may lead to destruction of normal tissue architecture, organ dysfunction, organ failure and eventually death. Fibrosis underpins the morbidity and mortality of, many serious (chronic) diseases of the lung, kidney, skin, liver, heart, etc., such as idiopathic lung fibrosis, chronic kidney disease and systemic scleroderma. Fibrosis is estimated to contribute to up to 45% of deaths worldwide. There is no cure. Existing solutions only relieve symptoms and insufficiently alter the course of these chronic progressive diseases. Thus, there is an urgent need to develop novel therapeutic strategies to treat these serious conditions.

Inhibitors according to the present invention may be selected from the group consisting of a polypeptide, a small molecule, an RNA, an antibody, and an antibody fragment. The invention further provides combinations of the inhibitors described herein. For example, it is envisaged that two, three, four, or five of the inhibitors of the invention may be comprised together in one composition or used together. The invention further provides compositions comprising the inhibitor(s) described herein. In embodiments where the inhibitor is an antibody, the antibody is selected from the group consisting of a monoclonal, polyclonal, bispecific, and chimeric antibody.

The inhibitors of the present invention may prevent TANGO1 heterodimerisation by targeting, interacting with, and/or binding to a region of TANGO1 which mediates interaction with cTAGE5, such as the CC2 region of TANGO1. The CC2 region of TANGO1 is known to dimerise with the CC2 region of cTAGE5 to form a TANGO1/cTAGE5 heterodimer. Targeting and/or binding to an interaction domain associated with this dimerisation process inhibits the formation of TANGO1/cTAGE5 heterodimers. The CC2 region of TANGO1 may have a sequence according to SEQ ID NO: 18. In some embodiments of the invention, the inhibitor may target a region of TANGO1 having high sequence identity to SEQ ID NO: 18, such as at least 75%, at least 80%, at least 85%, at least 90%, at least 95%, or at least 98% identity to SEQ ID NO: 18. The inhibitor may target and/or bind to a region within or a region identical to SEQ ID NO: 3. SEQ ID NO: 3 represents both the inhibitor P2 and a domain within the CC2 of TANGO1 which mediates interaction with both cTAGE5 CC2 and TALI CC2. SEQ ID NO 3 represents residues 30 to 59 of SEQ ID NO: 18. In some embodiments, the inhibitor of the invention targets and/or binds within a region starting from residue 20, 25, 28, 29, or 30, and ending at residue 69, 64, 61, 60, or 59, of SEQ ID NO: 18. In some embodiments, the inhibitor targets and/or binds to a region at least 5, at least 10, at least 15, at least 20, at least 25, or at least 30 residues in length. In some embodiments, the inhibitor targets and/or binds to a sequence according to SEQ ID NO: 3. The inhibitor may target and/or bind to a region within or a region identical to SEQ ID NO: 5. SEQ ID NO: 5 represents both the inhibitor P3 and a domain within the CC2 of TANGO1 which mediates interaction with both cTAGE5 CC2 and TALI CC2. SEQ ID NO 5 represents residues 60 to 89 of SEQ ID NO: 18. In some embodiments, the inhibitor of the invention targets and/or binds within a region starting from residue 50, 55, 58, 59, or 60, and ending at residue 99, 94, 91, 90, or 89, of SEQ ID NO: 18. In some embodiments, the inhibitor targets and/or binds to a region at least 5, at least 10, at least 15, at least 20, at least 25, or at least 30 residues in length. In some embodiments, the inhibitor targets and/or binds to a sequence according to SEQ ID NO: 5. In some embodiments, the inhibitor targets and/or binds to a sequence within the region starting from residue 30 and ending residue 89 of SEQ ID NO: 18.

The inhibitor of the invention targeting the CC2 region of TANGO1 may mimic the sequence of the CC2 region of cTAGE5 by having a polypeptide sequence similar or identical to that of the CC2 region of cTAGE5 (SEQ ID NO: 23). The inhibitor of the invention targeting the TANGO1 CC2 domain may comprise or consist of a portion of the polypeptide according to SEQ ID NO: 23, which corresponds to the CC2 domain of cTAGE5. In some embodiments, the inhibitor may comprise a polypeptide having high sequence identity to SEQ ID NO: 23. For example, the inhibitor may have at least 50%, at least 55%, at least 60%, at least 65%, at least 70%, at least 75%, at least 80%, at least 85%, at least 90%, at least 95%, or at least 98% identity to SEQ ID NO: 23.The inhibitor may comprise a polypeptide having high sequence identity to SEQ ID NO: 7 or SEQ ID NO: 9. In some embodiments, the inhibitor has at least 75%, at least 80%, at least 85%, at least 90%, at least 95%, or at least 98% identity to SEQ ID NO: 7 or SEQ ID NO: 9. In some embodiments, the inhibitor comprises or consists of a sequence identical to SEQ ID NO: 7 or SEQ ID NO: 9. In having a polypeptide sequence similar or identical to that of the CC2 region of cTAGE5 the inhibitor may bind to the CC2 region of TANGO1. SEQ ID NO: 7 represents both the inhibitor P4, and a region of the cTAGE5 CC2 domain. SEQ ID NO: 9 represents both the inhibitor P5, and a region of the cTAGE5 CC2 domain. The inhibitor of the invention may therefore prevent TANGO1 heterodimerisation with cTAGE5 through competitive inhibition of the TANGO1 CC2 binding site. Alternatively, an inhibitor of the invention may bind to the CC2 region of TANGO1 and may have an alternative sequence than that of SEQ ID NO: 7 or SEQ ID NO: 9. Alternatively, the inhibitor of the invention may bind to a different region of TANGO1 and prevent TANGO1/cTAGE5 heterodimerisation through non-competitive inhibition, or other conformational change of TANGO1. Alternatively, the inhibitor of the invention may bind to, target, or otherwise interact with any site of TANGO1 or cTAGE5 in order to inhibit heterodimerisation.

The inhibitor of the invention targeting the CC2 region of TANGO1 may mimic the sequence of the CC2 region of TALI by having a polypeptide sequence similar or identical to that of the CC2 region of TALI. The inhibitor of the invention targeting the TANGO1 CC2 domain may comprise or consist of a portion of the polypeptide according to SEQ ID NO: 23, which corresponds to the CC2 domain of TALI. In some embodiments, the inhibitor may comprise a polypeptide having high sequence identity to SEQ ID NO: 23. For example, the inhibitor may have at least 50%, at least 55%, at least 60%, at least 65%, at least 70%, at least 75%, at least 80%, at least 85%, at least 90%, at least 95%, or at least 98% identity to SEQ ID NO: 23.The inhibitor may comprise a polypeptide having high sequence identity to SEQ ID NO: 7 or SEQ ID NO: 9. In some embodiments, the inhibitor has at least 75%, at least 80%, at least 85%, at least 90%, at least 95%, or at least 98% identity to SEQ ID NO: 7 or SEQ ID NO: 9. In some embodiments, the inhibitor comprises or consists of a sequence identical to SEQ ID NO: 7 or SEQ ID NO: 9. In some embodiments, the inhibitor may comprise polypeptide comprising both SEQ ID NO: 7 and SEQ ID NO: 9. In having a polypeptide sequence similar or identical to that of the CC2 region of TALI the inhibitor may bind to the CC2 region of TANGO1. The inhibitor of the invention may therefore prevent TANGO1 heterodimerisation with TALI through competitive inhibition of the TANGO1 CC2 binding site. Alternatively, an inhibitor of the invention may bind to the CC2 region of TANGO1 and may have an alternative sequence than that of SEQ ID NO: 7 or SEQ ID NO: 9. Alternatively, the inhibitor of the invention may bind to a different region of TANGO1 and prevent TANGO1/TALI heterodimerisation through non-competitive inhibition, or other conformational change of TANGO1. Alternatively, the inhibitor of the invention may bind to, target, or otherwise interact with any site of TANGO1 or TALI in order to inhibit heterodimerisation.

The inhibitors of the present invention may prevent TANGO1 heterodimerisation by targeting, interacting with, and/or binding to a region of cTAGE5 which mediates interaction with TANGO1, such as the CC2 region of cTAGE5. The CC2 region of cTAGE5 is known to dimerise with the CC2 region of TANGO1 to form a TANGO1/cTAGE5 heterodimer. Targeting and/or binding to an interaction domain associated with this dimerisation process inhibits the formation of TANGO1/cTAGE5 heterodimers. The CC2 region of cTAGE5 may have a sequence according to SEQ ID NO: 23. In some embodiments of the invention, the inhibitor may target a region of cTAGE5 having high sequence identity to SEQ ID NO: 23, such as at least 75%, at least 80%, at least 85%, at least 90%, at least 95%, or at least 98% identity to SEQ ID NO: 23. The inhibitor may target and/or bind to a region within SEQ ID NO: 7 or SEQ ID NO: 9. S SEQ ID NO: 7 represents both the inhibitor P4, and a region of the cTAGE5 CC2 domain. SEQ ID NO: 9 represents both the inhibitor P5, and a region of the cTAGE5 CC2 domain. SEQ ID NO 7 represents residues 378 to 407 of SEQ ID NO: 21 and residues 39 to 68 of SEQ ID NO: 23. SEQ ID NO 9 represents residues 408 to 438 of SEQ ID NO: 21 and residues 69 to 99 of SEQ ID NO: 23. In some embodiments, the inhibitor of the invention targets and/or binds within a region starting from residue 29, 34, 37, 38, or 39, and ending at residue 78, 73, 70, 69, or 68, of SEQ ID NO: 23. In some embodiments, the inhibitor targets and/or binds to a region at least 5, at least 10, at least 15, at least 20, at least 25, or at least 30 residues in length. In some embodiments, the inhibitor targets and/or binds to a sequence according to SEQ ID NO: 7. In some embodiments, the inhibitor of the invention targets and/or binds within a region starting from residue 59, 64, 67, 68, or 69, and ending at residue 109, 104, 101, 100, or 99, of SEQ ID NO: 23. In some embodiments, the inhibitor targets and/or binds to a region at least 5, at least 10, at least 15, at least 20, at least 25, or at least 30 residues in length. In some embodiments, the inhibitor targets and/or binds to a sequence according to SEQ ID NO: 9. In some embodiments, the inhibitor targets and/or binds to a sequence within the region starting from residue 39 and ending residue 99 of SEQ ID NO: 23.

The inhibitor of the invention targeting the CC2 region of cTAGE5 may mimic the sequence of the CC2 region of TANGO1 by having a polypeptide sequence similar or identical to that of the CC2 region of TANGO1 (SEQ ID NO: 18). The inhibitor of the invention targeting the cTAGE5 CC2 domain may comprise or consist of a portion of the polypeptide according to SEQ ID NO: 18, which corresponds to the CC2 domain of TANGO1. In some embodiments, the inhibitor may comprise a polypeptide having high sequence identity to SEQ ID NO: 18. For example, the inhibitor may have at least 50%, at least 55%, at least 60%, at least 65%, at least 70%, at least 75%, at least 80%, at least 85%, at least 90%, at least 95%, or at least 98% identity to SEQ ID NO: 18. The inhibitor may comprise a polypeptide having high sequence identity to SEQ ID NO: 3 or SEQ ID NO: 5. In some embodiments, the inhibitor has at least 75%, at least 80%, at least 85%, at least 90%, at least 95%, or at least 98% identity to SEQ ID NO: 3 or SEQ ID NO: 5. In some embodiments, the inhibitor comprises or consists of a sequence identical to SEQ ID NO: 3 or SEQ ID NO: 5. In some embodiments, the inhibitor may comprise polypeptide comprising both SEQ ID NO: 3 and SEQ ID NO: 5. In having a polypeptide sequence similar or identical to that of the CC2 region of TANGO1 the inhibitor may bind to the CC2 region of cTAGE5. SEQ ID NO: 3 represents both the inhibitor P2, and a region of the TANGO1 CC2 domain. SEQ ID NO: 5 represents both the inhibitor P3, and a region of the TANGO1 CC2 domain. The inhibitor of the invention may therefore prevent TANGO1 heterodimerisation with cTAGE5 through competitive inhibition of the cTAGE5 CC2 binding site. Alternatively, an inhibitor of the invention may bind to the CC2 region of cTAGE5 and may have an alternative sequence than that of SEQ ID NO: 3 or SEQ ID NO: 5. Alternatively, the inhibitor of the invention may bind to a different region of cTAGE5 and prevent TANGO1/cTAGE5 heterodimerisation through non-competitive inhibition, or other conformational change of cTAGE5. Alternatively, the inhibitor of the invention may bind to, target, or otherwise interact with any site of TANGO1 or cTAGE5 in order to inhibit heterodimerisation.

The inhibitors of the present invention may prevent TANGO1 heterodimerisation by targeting and/or binding to a region of TANGO1 which mediates interaction with the NRZ tether complex, such as the CC1 region of TANGO1. The CC1 region of TANGO1 is known to recruit the NRZ tether complex. Targeting and/or binding to an interaction domain associated with this recruitment process inhibits the formation of a collagen carrier. The CC1 region of TANGO1 may have a sequence according to SEQ ID NO: 17. In some embodiments of the invention, the inhibitor may target a region of TANGO1 having high sequence identity to SEQ ID NO: 17, such as at least 75%, at least 80%, at least 85%, at least 90%, at least 95%, or at least 98% identity to SEQ ID NO: 17. The inhibitor may target and/or bind to a region within or a region identical to SEQ ID NO: 1. SEQ ID NO: 1 represents the inhibitor P1. SEQ ID NO 1 represents residues 43 to 72 of SEQ ID NO: 17. In some embodiments, the inhibitor of the invention targets and/or binds within a region starting from residue 33, 38, 41, 42, or 43, and ending at residue 82, 77, 74, 73, or 72, of SEQ ID NO: 17. In some embodiments, the inhibitor targets and/or binds to a region at least 5, at least 10, at least 15, at least 20, at least 25, or at least 30 residues in length. In some embodiments, the inhibitor targets and/or binds to a sequence according to SEQ ID NO: 1.

The inhibitor of the invention may target the NRZ tether complex and may comprise or consist of a portion of the polypeptide according to SEQ ID NO: 17, which corresponds to the CC1 domain of TANGO1. In some embodiments, the inhibitor may comprise a polypeptide having high sequence identity to SEQ ID NO: 17. For example, the inhibitor may have at least 50%, at least 55%, at least 60%, at least 65%, at least 70%, at least 75%, at least 80%, at least 85%, at least 90%, at least 95%, or at least 98% identity to SEQ ID NO: 17.

The inhibitor of the invention may target and/or bind to the proteins NBAS, RINT1, and/or ZW10 of the NRZ tether complex. The inhibitor may comprise a polypeptide having high sequence identity to SEQ ID NO: 1. In some embodiments, the inhibitor has at least 75%, at least 80%, at least 85%, at least 90%, at least 95%, or at least 98% identity to SEQ ID NO: 1. In some embodiments, the inhibitor comprises or consists of a sequence identical to SEQ ID NO: 1. In having a polypeptide sequence similar or identical to that of the CC1 region of TANGO1 the inhibitor may bind to the proteins NBAS, RINT1, and/or ZW10 of the NRZ tether complex. SEQ ID NO: 1 represents both the inhibitor P1, and a region of the TANGO1 CC1 domain. The inhibitor of the invention may therefore prevent TANGO1-mediated recruitment of ERGIC membranes. Alternatively, an inhibitor of the invention may bind to one or more of the NRZ complex proteins NBAS, RINT1, and ZW10 and may have an alternative sequence than that of SEQ ID NO: 1. Alternatively, the inhibitor of the invention may bind to, target, or otherwise interact with any site of TANGO1 or the NRZ complex proteins NBAS, RINT1, and ZW10 in order to inhibit TANGO1-mediated ERGIC membrane recruitment.

The inhibitors, or compositions comprising the inhibitor(s), of the invention may further be provided with signal sequences. The signal sequences may assist the inhibitors and/or compositions in penetrating cell membranes. The signal sequences may direct the inhibitors and/or compositions to the target organ, tissue, and/or cell(s). The signal sequences may tag the inhibitors and/or compositions such that they may be identified. The signal sequences may assist the inhibitors and/or compositions in being trafficked in the intracellular environment. The signal sequences may assist the inhibitors and/or compositions to fold correctly *in vivo.* The signal sequences may assist the inhibitors and/or compositions to assemble with other inhibitors and/or compositions *in vivo.* The signal sequences may assist modular units of inhibitors and/or compositions according to the invention to assemble into inhibitors and/or compositions of the invention *in vivo.* The signal sequences may be used to enhance the stability of the inhibitors. The signal sequences may be peptides, covalent peptide tags, protein tags, nucleotides, antibodies, antibody fragments, antigens, solubilisation tags, fluorescent tags, and/or epitope tags. The signal sequences may be appended to the C-terminus, N-terminus, or an internal location of a polypeptide. In some embodiments, the signal sequences may be one or more signals selected from the group consisting of SEQ ID NOs: 13, and 30 to 36. In some embodiments, the signal sequences may be one or more sequences encoding polypeptides from the group consisting of SEQ ID NOs: 13, and 30 to 36.

The inhibitor of the invention may comprise an RNA molecule designed to inhibit TANGO1 heterodimerisation or TANGO1-mediated recruitment of recruitment of ER-Golgi intermediate compartment (ERGIC) membranes. The RNA may be selected from the group consisting of shRNA, siRNA, miRNA, antisense oligonucleotides, RNA aptamers, modified RNA, native RNA, and combinations thereof. The RNA may inhibit TANGO1 heterodimerisation or TANGO1-mediated recruitment of recruitment of ERGIC membranes through targeted knock down of TANGO1, cTAGE5, and/or TALI protein expression. In some embodiments, the antisense oligonucleotide is between 7 and 15 nucleotides long, and is a locked nucleic acid In some embodiments, the antisense oligonucleotide is between 18 and 25 nucleotides long, and is a 2'-O-methyl phosphorothioate ribonucleic acid. In some embodiments, the antisense oligonucleotide is between 18 and 25 nucleotides long, and is a 2'-O-methoxyethyl-modified phosphorothioate ribonucleic acid. In some embodiments, the antisense oligonucleotide has least one cytosine residue within the oligonucleotide which is methylated.

The invention further provides vectors carrying or encoding inhibitors described herein. The vectors may be selected from the group consisting of plasmid, adenovirus, adeno-associated virus (AAV), lentivrus (LV), cytomegalovirus (CMV), herpes simplex virus (HSV), vesicular stomatitis virus (VSV), and baculovirus. The invention further provides an expression cassette encoding the inhibitors described herein. The invention further provides an mRNA encoding inhibitors described herein. Such mRNA may be comprised within an RNA vaccine, (synthetic) circular RNA, an RNA nanostructure, and/or an RNA/DNA nanostructure.

The invention provides delivery vehicles comprising the inhibitor(s), compositions vectors, and/or expression cassettes described herein. Such delivery vehicles may be selected from the group consisting of a nanoparticle, a vesicle, a nanovesicle, a lipid nanoparticle, a lipid nanovesicle, a liposome, and an exosome. In some embodiments the delivery vehicle is provided with a signal sequence or tag.

The invention further provides pharmaceutical compositions and therapeutic agents comprising the inhibitor(s), compositions, vectors, expression cassettes, and/or delivery vehicles described herein. The pharmaceutically acceptable composition may comprise a pharmaceutically acceptable excipient. In some embodiments, the pharmaceutically acceptable composition is formulated for topical administration. In some embodiments, the pharmaceutically acceptable excipient comprises or consists of one or more selected from the group consisting of glycerin, propylene glycol, dimethicone, squalane, shea butter, cocoa butter, carbomer, xanthan gum, cetyl alcohol, stearyl alcohol, cellulose derivatives, methylparaben, propylparaben, phenoxyethanol, propylene glycol, dimethyl sulfoxide, vitamin e, ascorbic acid, fragrance oils, colorants, polysorbate 80, sodium lauryl sulfate, acrylates/c10-30 alkyl acrylate crosspolymer, polyvinylpyrrolidone, hydroxyethyl urea, ethanol, and isopropyl alcohol. The pharmaceutical composition and/or therapeutic agent may be provided as a lotion, soap, ointment, eye drop, patch, dressing, gauze, cream, gel, or foam.

The invention provides the inhibitor(s), compositions, vectors, expression cassettes, and/or delivery vehicles described herein for use in medicine. The invention further provides the pharmaceutical compositions and/or therapeutic agents described herein for use in methods of treatment of a disease or disorder. The invention therefore encompasses methods of treatment of a disease or disorder, wherein the method comprises administering the pharmaceutical compositions and/or therapeutic agents described herein to a patient in need thereof. In some embodiments, the disease or disorder is a fibrotic disease or disorder. In some embodiments the disease or disorder is selected from the group consisting of scleroderma, local scleroderma, dermatofibrosis lenticularis disseminata, morphea, keloids, peyronie's disease, nephrogenic systemic fibrosis, scarring, idiopathic pulmonary fibrosis, non-idiopathic pulmonary fibrosis, chronic kidney disease, systemic scleroderma skin fibrosis, hypertrophic scars, liver fibrosis, and cardiovascular disease. In some embodiments, the disease or disorder is selected from the group consisting of scleroderma, dermatofibrosis lenticularis disseminata, morphea, keloids, peyronie's disease, nephrogenic systemic fibrosis, and scarring.

The invention further provides the pharmaceutical compositions and/or therapeutic agents described herein for use in methods of treating or managing a wound. The invention therefore encompasses methods of a treating or managing a wound, wherein the method comprises administering the pharmaceutical compositions and/or therapeutic agents described herein to a patient in need thereof. In some embodiments, the method comprises administering pharmaceutical compositions and/or therapeutic agents described herein to a wound.

In some embodiments, the pharmaceutical compositions and/or therapeutic agents may be administered topically, orally, intravenously, intramuscularly, subcutaneously, transdermally, inhalationally, and/or rectally.

In some embodiments, the pharmaceutical compositions and/or therapeutic agents prevent or reduce scarring. As such, the pharmaceutical compositions and/or therapeutic agents may be used in a method of preventing or reducing scarring in a subject in need thereof. Such a method may comprise topical administration of the pharmaceutical compositions and/or therapeutic agents. Such topical administration may be five times daily, four times daily, three times daily, twice daily, daily, every two days, every three days, every four days, every five days, every six days, weekly, fortnightly, or monthly.

The invention also provides the pharmaceutical compositions and/or therapeutic agents for use in the treatment of diseases associated with fibrillin and/or fibronectin export, the method comprising administering the pharmaceutical compositions and/or therapeutic agents to a subject in need thereof.

Pharmaceutical preparations of the invention are formulated to conform with regulatory standards and can be administered orally, intra-venously, topically, or via other standard routes. The pharmaceutical preparations may be in the form of tablets, pills, edible films, lotions, gels, liquids, powders, suppositories, suspensions, liposomes, microparticles or other suitable formulations known in the art.

The inhibitors, compositions, pharmaceutical compositions, and therapeutic agents described herein may all be used in cosmetic methods, such as for treating scarring or a skin blemish.

The invention further provides a method of reducing collagen export from the endoplasmic reticulum of a cell, the method comprising contacting the inhibitor(s), compositions, vectors, expression cassettes, delivery vehicles, pharmaceutical compositions, and/or therapeutic agents with the cell.

Screening of molecules and proteins for binding to TANGO1, cTAGE5, TALI and/or NRZ tether complex proteins can be performed via automated high-throughput screening procedures. Hence, the invention provides methods for identifying inhibitor molecules via detection of a positive binding interaction between TANGO1, cTAGE5, TALI and/or NRZ tether complex proteins and a target molecule. Further screening steps may be used to determine whether the identified positive binding interaction is of pharmacological importance - i.e. whether the target molecule is capable of moderating TANGO1 heterodimerisation, bioactivity, and/or function. If a molecule with an inhibitory effect is identified, the molecule is classified as a 'hit' and can then be assessed as a potential candidate inhibitor. Additional factors may be taken into consideration at this time or before, such as the absorption, distribution, metabolism and excretion (ADME), bio-availability and toxicity profiles of the molecule, for example. If the potential drug molecule satisfies the pharmacological requirements, suitable compositions can be formulated for testing the activity *in vitro* and *in vivo,* in accordance with standard procedures known in the art.

### Examples

The invention will now be further illustrated by way of the following non-limiting examples.

Unless otherwise indicated, commercially available reagents and standard techniques in molecular biological and biochemistry were used.

Membrane permeant peptide inhibitors were designed that specifically target and disrupt the primary interface between TANGO1 and cTAGE5, an interaction that is required for collagen export from endoplasmic reticulum exit sites (ERES). Dissociation of the TANGO1 and cTAGE5 complex leads to their partial degradation and a consequent inhibition in the secretion of several ECM components, including collagens. Peptide inhibitor treatment in zebrafish resulted in altered tissue architecture and reduced granulation tissue formation during cutaneous wound healing. The inhibitors reduced secretion of several ECM proteins, including collagens, fibrillin and fibronectin in human dermal fibroblasts and in cells obtained from patients with a generalized fibrotic disease (scleroderma). Taken together, this targeted interference in the TANGO1-cTAGE5 binding interface enables therapeutic modulation of ERES function in ECM hypersecretion, during wound healing and fibrotic processes.

### Example 1 - Peptides inhibit TANGO1-cTAGE5 heterodimerization

Prior studies have revealed that TANGO1 heterodimerization with its paralog cTAGE5 is mediated by a coiled-coil region (shown as CC2 in the two proteins, Fig. 1A). AlphaFold2 was used to predict the structure of the TANGO1-cTAGE5 interface (T1-CC2-c5-CC2). The primary sequence used in the predictions is indicated by the amino acid numbering, corresponding to the numbers on Uniprot (Uniprot IDs, TANGO1: Q5JRA6, cTAGE5: Q96PC5-7). According to the predictions, the coiled-coil regions dimerize along their entire length.

Based on these structures, short polypeptides were designed corresponding to the primary sequences of these coiled-coil regions of TANGO1 and cTAGE5, which should competitively inhibit the TANGO1-cTAGE5 function (Fig. 1B). Two peptides were designed, named P2 and P5, of 30 or 31 amino acids respectively, conjugated to a cell penetrating dodecapeptide (shown in bold) to translocate the attached peptides into the cytoplasm P2: LRQKVEILNELYQQKEMALQKKLSQEEYER**GLRKRLRKFRNK** (SEQ ID NO: 4) and P5: LEKEEKLSKVDEKISHATEELETYRKRAKDL**GLRKRLRKFRNK** (SEQ ID NO: 10). This stretch of amino acids in TANGO1 and cTAGE5 are well conserved, showing high identity even in zebrafish cTAGE5. As controls, amino acids of P2 and P5 were scrambled to generate scrP2: VEEAKLYQRQSRMENLLEKEKQELLQKQIYGLRKRLRKFRNK (SEQ ID NO: 11) and scrP5: KLRKHELERLKSELEEIEATETKDYVADKKSGLRKRLRKFRNK (SEQ ID NO: 12).

To test whether peptides inhibit TANGO1-cTAGE5 heterodimerization in cells, a bioluminescence resonance energy transfer (NanoBRET)-based assay was employed, which uses Luciferase (NanoLuc) as the BRET energy donor and HaloTag protein labeled with the HaloTag 618 fluorescent ligand as the energy acceptor, to measure the interaction of two binding partners in live cells (Fig. 1C). HEK293T cells were transfected with a portion of the coiled-coil region (CC2) of TANGO1 with an N-terminal fusion of HaloTag (Halo-T1-CC2 - SEQ ID NO: 37) and the corresponding predicted binding region of cTAGE5 coiled-coil 2 or C5-CC2 with a Nanoluciferase (Nanoluc) tag at the N-terminus (NanoLuc-cTAGE5-CC2 - SEQ ID NO:38). Adding the HaloTag ligand showed a robust fluorescent signal, confirming that the coiled-coil regions do indeed interact under these conditions. Inhibiting the dimerization of the coiled-coil regions would induce a loss of fluorescence, which can be monitored readily. Cells expressing Halo-TANGO1-CC2 and NanoLuc-cTAGE5-CC2 were incubated in HaloTag 618 ligand and then treated with varying concentrations of peptide. Adding peptides P2, P5 or P2+P5 (0-200µM) reduced the emitted fluorescence, confirming both that the peptides were entering the cells, and that they inhibited TANGO1-CC2-cTAGE5-CC2 hetero-dimerization.

Levels of TANGO1 and cTAGE5 were quantified by Western blotting in control cells and those treated with peptides P2 (100µM), P5 (100µM), and P2+P5 (100µM each). Both TANGO1 and cTAGE5 protein levels were reduced in the presence of P2 and P5 (Fig. 1D) suggesting that targeting the TANGO1/cTAGE5 heterodimerization interface leads to unstable monomers.

### Example 2 - Peptides inhibit TANGO1/cTAGE5 in zebrafish

To test whether peptides inhibit TANGO1/cTAGE5 in vivo, zebrafish embryos from the 2-cell stage were treated with a combination of P2 and P5 (2µM or 4µM, each). Embryos were continuously treated with the peptides until 3 days post fertilization (dpf), when they were imaged. At 3 dpf, larvae treated with peptides had a shorter body axis (Fig. 2A), a phenotype also seen in TANGO1/cTAGE5 zebrafish knockouts. Some fish also presented with additional phenotypes reminiscent of defects associated with TANGO1/cTAGE5 knockouts 16, including pooled/clotted blood, darkened expanded yolk, and blistered skin (Fig.2B). Phenotypic changes appeared more frequently in larvae treated with P2+P5 at 4µM supporting a dose-dependent effect of the treatment (Fig. 2B).

TANGO1 and cTAGE5 have been implicated in collagen secretion. To evaluate changes in extracellular collagen architecture after peptide treatment, second-harmonic generation (SHG) microscopy was utilised to visualize collagen fibers in the larval tail. Zebrafish were fixed, de-yolked, and imaged using SHG microscopy in the tail blade, a region with a high density of organized collagen fibers. Second harmonic images were acquired from three areas of the tail (data not shown). Images of collagen fibers from all three areas suggested that control animals had a homogenous distribution in length and orientation, such that most fibers were well aligned with respect to their neighboring fibers. On the other hand, fibers in peptide-treated tails were considerably more heterogeneous in size and their relative alignment (quantified as 'dispersion' in Fig. 2C).

The altered ECM and changes in tissue architecture in peptide-treated zebrafish larvae are consistent with an inhibition of cTAGE5 and TANGO1 in vivo.

### Example 3 - Peptides bring about collagen retention in the ER

If the peptides inhibit TANGO1 or cTAGE5, they should reduce collagen export from the ER. The peptides were tested in two cell lines U2OS cells (collagen I) and RDEB/FB/C7 fibroblasts (collagen VII). Cells were incubated in serum-free medium containing ascorbic acid (250µM) and sodium ascorbate (1mM) for 20h to promote procollagen export from the ER.

Control U2OS cells or cells treated with peptide inhibitors (P2, P5, and P2+P5 at 100µM each) were fixed and processed for immunofluorescence microscopy to visualize intracellular collagen - quantification of which is shown in Fig. 3A. We observed increased collagen I in peptide-treated cells, which colocalized with the ER-resident protein Calnexin, consistent with the peptides inhibiting TANGO1-dependent ER export of collagens. In RDEB/FB/C7 cells also peptide incubation resulted in a retention of collagen VII in the ER as marked by Calnexin - quantification of which is shown in Fig. 3B.

RDEB/FB/C7 cells treated with peptides show TANGO1 recruitment to accumulations of collagen. TANGO1 puncta are colocalized with ERES markers Sec31A (data not shown), confirming that peptide inhibitors do not affect TANGO1 localization to ERES or its recruitment of collagen.

### Example 4 - Peptides inhibit collagen secretion in primary fibroblasts

To test if the peptides efficiently reduce collagen secretion also in primary cells derived from human individuals, normal human skin fibroblasts, the key producers of collagen in connective tissues, were treated with the peptides P2 and P5. Fluorescently-labeled peptides P2 and P5 entered cells readily, either individually or in combination, localizing throughout the cells (data not shown). Under these conditions, neither P2 nor P5 nor P2+P5 exerted cytotoxic effects as determined in an LDH release cytotoxicity assay (Fig. 4A) and cells showed similar morphology and proliferation rates. No significant intracellular accumulation of procollagen I was noted (Fig. 4B). The addition of P2+P5 resulted in a more than 60% reduction in secreted levels of procollagen I (Fig. 4C). Taken together, the data suggest that peptide treatment effectively inhibits collagen secretion. In addition, they also suggest that these primary cells can clear most accumulated intracellular collagen.

Whether the effect exerted by P2+P5 on primary human skin fibroblasts is reversible, and how long it lasts, was investigated. Fibroblasts were first treated (or not, serving as controls) with P2+P5 as earlier, and then supplied with growth-medium containing serum for 24, 48, or 72h in the absence of peptides. Supernatants showed a gradual increase in collagen I levels after the addition of serum (not shown), as well as a very strong reduction by P2+P5 after 24h (Fig. 4D). Levels of secreted collagen I after 48h were only mildly lower than untreated controls, and after 72h, the reduction was no longer detectable. These results indicate that P2+P5 inhibit collagen secretion for at least 24h after their removal and then their effect declines.

### Example 5 - TANGO1/cTAGE5 inhibition reduces the secretion of multiple ECM proteins

Given that fibroblasts produce several types of collagen, including collagen I, III and V, all of which contribute to the formation of heterotypic collagen I/III/V fibrils present in the large fibrillar network that characterizes the dermal ECM, the supernatants from control and P2+P5-treated fibroblasts were analayzed. Mass spectrometric quantification of secreted cargoes (the secretome) confirm significant reduction of the α1 and α2 chains of collagen I (COL1A1 and COL1A2), replicating previous immunoblot results. A significant reduction was observed in secreted collagen III (COL3A1), collagen V (COL5A1), collagen XII (COL12A1), the latter often found decorating the surface of collagen I/II/V fibrils, and collagen VI (COL6A2 and COL6A3), the structural component of pericellular beaded filaments. Interestingly, interfering with TANGO1 activity also impaired the secretion of some non-collagen ECM proteins that were previously not known to be TANGO1-cargoes, such as the core protein of the proteoglycan versican (VCAN), fibrillin1 (FBN1), a structural component of fibrillin-microfibrils, fibronectin, or laminin subunit α4 (LAMA4), a component of basement membranes. There were no detected ECM proteins that were hypersecreted after treatment with P2+P5. Overall, there were fewer hypersecreted proteins than hypo-secreted. These results are presented in Table 1. The highest apparent increase was MIA3 (TANGO1) itself, which is merely detection of the added peptides derived from TANGO1. Gene ontology (GO) analysis showed that the proteins primarily affected by peptide treatment include ECM structural components, particularly collagen (Fig. 5). Several cargoes showed no significant change in secretion after treating cells with the peptides (Table 2), highlighting the specificity / selectivity of peptide activity.

**Table 1**

| **Gene names** | **Protein IDs** | **Protein names** | **-Log₁₀ T test P-value (P2P5 vs. Control)** | **Log₂ FC (P2P5 vs. Control)** |
|---|---|---|---|---|
| VCAN | P13611 | Versican core protein | 1,81170 | -5,19527 |
| FBN1 | P35555 | Fibrillin-1 | 4,01801 | -4,67462 |
| COL12A1 | Q99715 | Collagen alpha-1(XII) chain | 4,37419 | -4,67456 |
| COL3A1 | P02461 | Collagen alpha-1(III) chain | 3,92008 | -3,59692 |
| COL5A1 | P20908 | Collagen alpha-1(V) chain | 3,42365 | -3,12562 |
| COL6A3 | P12111 | Collagen alpha-3(VI) chain | 3,13966 | -3,01714 |
| CTSB | P07858 | Cathepsin B | 2,05424 | -2,54762 |
| COL6A2 | P12110 | Collagen alpha-2(VI) chain | 1,67596 | -2,31061 |
| LAMA4 | Q16363 | Laminin subunit alpha-4 | 2,61542 | -2,25661 |
| CLU | P10909 | Clusterin | 1,86799 | -2,23954 |
| PDIA3 | P30101 | Protein disulfide-isomerase A3 | 1,95555 | -2,19724 |
| C1S | P09871 | Complement C1s subcomponent | 1,90802 | -1,85249 |
| FSTL1 | Q12841 | Follistatin-related protein 1 | 3,09804 | -1,83823 |
| PSAP | P07602 | Prosaposin | 2,03952 | -1,74644 |
| TMSB4X | P62328 | Thymosin beta-4 | 1,80659 | -1,60465 |
| COL1A1 | P02452 | Collagen alpha-1(I) chain | 3,47620 | -1,55965 |
| YWHAZ | P63104 | 14-3-3 protein zeta/delta | 2,41210 | -1,47430 |
| RCN1 | Q15293 | Reticulocalbin-1 | 1,71327 | -1,36175 |
| COL1A2 | P08123 | Collagen alpha-2(I) chain | 4,02213 | -1,33409 |
| MIA3 | Q5JRA6 | Melanoma inhibitory activity protein 3 | 1,66826 | 8,49901 |
| ACTB | P60709 | Actin | 2,92456 | 1,91751 |
| ENO1 | P06733 | Alpha-enolase | 2,38767 | 1,85727 |
| CFL1 | P23528 | Cofilin-1 | 2,54660 | 1,63683 |
| LGALS1 | P09382 | Galectin-1 | 3,19137 | 1,63093 |
| PGK1 | P00558 | Phosphoglycerate kinase 1 | 1,69194 | 1,40227 |
| SERPINE1 | P05121 | Plasminogen activator inhibitor 1 | 3,14065 | 1,33331 |
| VIM | P08670 | Vimentin | 2,92202 | 1,31643 |

**Table 2**

| **Gene names** | **Protein IDs** | **Protein names** | **-Log₁₀ T test P-value (P2P5 vs. Control)** | **Log₂ FC (P2P5 vs. Control)** |
|---|---|---|---|---|
| MMP1 | P03956 | Interstitial collagenase | 1,70477 | 0,83577 |
| SERPINE2 | P07093 | Glia-derived nexin | 1,24836 | 0,71191 |
| TIMP1 | P01033 | Metalloproteinase inhibitor 1 | 1,07280 | 0,59529 |
| SPARC | P09486 | SPARC | 1,24367 | 0,48131 |
| ANXA2 | P07355 | Annexin A2 | 0,59280 | 0,41588 |
| TGFBI | Q15582 | Transforming growth factor-beta-induced protein iq-h3 | 1,21532 | 0,41117 |
| CYR61 | O00622 | Protein CYR61 | 0,73704 | 0,29120 |
| FBLN2 | P98095 | Fibulin-2 | 0,70771 | 0,27581 |
| CST3 | P01034 | Cystatin-C | 2,50978 | 0,26645 |
| TIMP2 | P16035 | Metalloproteinase inhibitor 2 | 0,14051 | 0,18951 |
| MMP2 | P08253 | 72 kDa type IV collagenase | 0,21836 | 0,16330 |
| PKM | P14618 | Pyruvate kinase PKM | 0,05721 | 0,04434 |
| CCDC80 | Q76M96 | Coiled-coil domain-containing protein 80 | 0,00974 | 0,00646 |
| COL6A1 | P12109 | Collagen alpha-1(VI) chain | 0,07256 | -0,05182 |
| LUM | P51884 | Lumican | 0,89805 | -0,21413 |
| POSTN | Q15063 | Periostin | 0,35686 | -0,38590 |
| SOD1 | P00441 | Superoxide dismutase [Cu-Zn] | 0,00000 | -0,57423 |
| ECM1 | Q16610 | Extracellular matrix protein 1 | 1,65417 | -0,88092 |

### Example 6 - Controlled inhibition of wound healing in zebrafish

An important test of the inhibitors is whether they show efficacy in controlling collagen deposition into the granulation tissue during the wound healing process *in vivo.* Therefore the effect of the peptides on the healing of laser-induced cutaneous full-thickness in adult zebrafish was studied. In such wounds, granulation tissue is formed starting at two days post wounding (2 dpw) and reaching maximal sizes at 4 dpw 28. Fish wounds were microinjected both at 2 and at 3 dpw with PBS (control) or peptide (4µM P2+P5 in PBS).

At 4 dpw, histology and anti-type I collagen immunofluorescence labelling with DAPI-counterstaining of cell nuclei revealed that peptide treatment resulted in reduced granulation tissue formation (Fig. 6A) and reduced collagen deposition (Fig 6B) within the granulation tissue, while the density of cells / fibroblasts was slightly increased. In addition, TUNEL staining of dying cells did not reveal differences between controls and peptide-treated samples (data not shown).

### Example 7 - Peptides inhibit collagen secretion in activated healthy and in patient-derived primary fibroblasts

Whether the identified peptides can reduce pathologically increased collagen secretion, such as that seen in activated fibroblasts and in fibroblasts derived from patients with scleroderma was assessed. Activation was accomplished by treatment of fibroblasts with TGFβ, which prominently enhances collagen I production. Incubating TGFβ-activated skin fibroblasts from healthy individuals with P2+P5 showed a clear inhibition of collagen secretion by the combination of P2+P5 (Fig. 7A). The inhibitory effects were also clearly visible in scleroderma fibroblasts, especially after TGFβ activation (Fig.7B). Most promisingly, both healthy and scleroderma fibroblasts showed reduced secretion of other abundant fibrotic ECM components, fibrillin1 and fibronectin (Figs. 7A & B), further underscoring the importance of TANGO1 in controlling pathophysiological secretion of bulky ECM secretory cargoes.

### Example 8 - B: Peptides ameliorate skin fibrosis in a murine model

The K14-Snail-Tg mouse was developed as described earlier as a model of skin fibrosis (Manando Nakasaki,et.al; Neha Pincha,et.al). All mice were housed in the Animal Care and Resource Centre of the Bangalore Life Science Cluster under specific pathogen-free conditions. Animal work was approved by the inStem Institutional Animal Ethics Committee following the norms specified by the Committee for the Purpose of Control and Supervision of Experiments on Animals (Government of India). WT and Snail transgenic mice were injected subcutaneously in the back skin with 100 µl of 20µM of TANGO1/cTAGE5 inhibitors (P2 (SEQ ID NO: 4), P5 (SEQ ID NO: 10), and P2+P5) dissolved in water and 100 µl of water as vehicle control. The injections were administered weekly for eight weeks starting at postnatal day 7. In the 9th week, the mice were sacrificed and back skin was harvested from the injected region and embedded in tissue freezing medium (OCT, Leica). 30 µm thick sections were generated with a cryostat and stained with Hematoxylin (Sigma C.I. 75290) and Eosin (Sigma E4009-25G). Dermal thickness was measured as the average area of multiple 300 µm wide sections of the tissue using imaged software. Sebaceous hyperplasia was used as a landmark of a phenotypic skin in the Snail transgenic mice since the cutaneous phenotype in this transgenic mouse is mosaic. To avoid sectioning artifacts, only areas around full length hair follicles were analysed for dermal thickness measurements. In addition, measurements between wild type and Snail transgenic skin were matched for sections that were in the same stage of the hair cycle. Quantification is the average of at least four sections each from three biological replicates.

### Results 1: P2 and P5

We assessed whether these peptides are capable of reducing the fibrotic phenotype in a mouse model of skin fibrosis (REF). This mouse model is based on the transgenic expression of the transcription factor Snail in the epidermal keratinocytes, which recapitulates many of the cardinal features of scleroderma including dermal fibrosis. Similar to the human disease, the fibrotic phenotype of the Snail mouse model is progressive, such that adult mice exhibit maximal dermal fibrosis. Consequently, we used neonatal animals and injected the peptides weekly, over a two-month period. Following this treatment regimen, dermal thickness was measured as a proxy for fibrosis in the dermal compartment of the skin in mice injected with peptides versus vehicle control (water). Consistent with our previous observation with the Snail transgenic skin (REF), Snail mice injected with vehicle control had ~2.5 fold thicker dermis than its wild type counterpart (Fig. 8A). The combination of peptides resulted in a reduction in fibrosis of approximately 25%.

### Results 2: P1

The therapeutic efficacy of the peptide inhibitor P1 was evaluated by its ability to inhibit collagen secretion in connection to relief of disease symptoms. The Snail mouse model of scleroderma was used. This mouse model is developed via the transgenic expression of the transcription factor Snail in the basal keratinocytes of the epidermis. Mice originating from this strain, were treated with P1 (SEQ ID NO: 2), and control peptide (P6; only the membrane-permeable sequence (SEQ ID NO: 13) for 4 weeks with 2-3 days interval (10 µM s.c. for each treatment day). After four weeks, the mice were sacrificed and skin at the peptide and an untreated site was dissected. Clinical signs of scleroderma, i.e. skin erythema, skin thickening and scaling and excessive deposition of collagen, and histopathology and quantification of cytokines in mouse skin biopsies, were scored.

Results show that P1 effectively reduced thickening of the skin, while P6 had a less pronounced effect (Fig. 8B).

### Materials and Methods

### Peptide synthesis

Peptides were synthesized by automated solid-phase peptide synthesis (SPPS) (peptide synthesizer from MultiSynTech, Syro I) using the Fmoc (fluorenylmethoxycarbonyl)/t-Bu (tert-butyl) strategy on Rinkamid resin (loading 0.48 mmol, 0.015 mmol scale). Amino acids were coupled using 8 equivalents (eq) N,N'-diisopropylcarbodiimide (DIC) and ethyl cyanohydroxyiminoacetate (Oxyma) in dimethylformamide (DMF) as activating reagents. Fmoc was cleaved off using 20 % piperidine in DMF. Finally, the resin was washed with dichloromethane (DCM), methanol (MeOH) and diethyl ether (Et2O), respectively.

For cell uptake and fluorescence microscopy studies, also 5,6-carboxyfluorescein (CF)-labeled peptides were synthesized. Therefore, 5 eq CF, Oxyma and DIC were applied and peptides were labeled at the N-terminal while still attached to the resin.

After complete synthesis, peptides were cleaved by treating the resin for 3 h at room temperature with a mixture of concentrated (conc.) trifluoroacetic acid (TFA)/triisopropylsilane (TIS)/H2O (95:2.5:2.5, v/v/v). Subsequently, peptides were precipitated in ice-cold diethyl ether. Reversed-phase (RP) RP-high pressure liquid chromatography (HPLC) (column: CC 125/4.6 Nucleodur 100-5 C18e (Macherey-Nagel) using a gradient from 10-60 % of acetonitrile (ACN) in H2O with 0.1 % formic acid for sample separation, followed by electrospray ionization-mass spectrometry (ESI-MS, Thermo Scientific LTQ-XL) was used for identification. Purification was achieved by semi-preparative RP-HPLC (Hitachi Elite LaChrom) on a VP 250/8 Nucleodur 100-5 C18e column (Macherey-Nagel) using linear gradients from 10-60 % B in A (A= 0.1 % conc. TFA in water; B=0.1 % conc. TFA in ACN) over 45 min and a flow rate of 1.5 ml/min. Final purity of all compounds was >95 %. Finally, to exchange the remaining TFA by chloride peptides were diluted in 3 mL 80 mM HCl solution, incubated for 2 h shaking, and lyophilized overnight. This procedure was repeated two times.

### NanoBRET

The indicated portions of the coiled-coil regions of TANGO1 were amplified by PCR and cloned into pHTN HaloTag^{®} CMV-neo (Promega) by Gibson assembly. The cTAGE5 (c5-CC2) was cloned by Gibson assembly into the pNLF-N CMV hygro vector (promega). The vectors used were as described earlier
To test for protein-protein interaction between TANGO1-CC2 and c5-CC2, we plated 1.4x105 cells per well in a 24-well plate. Cells were transfected with 500 ng of pHTN-TANGO1-CC2, 5 ng of pNLFN-cTAGE5-CC2, 0.9 µl of Lipofectamine 3000 and 1 µl of P3000 Reagent (Thermo Fisher Scientific).

To test various concentrations of peptides to inhibit PPI interaction, we use 6 well plates, with 8×10⁵ cells per well. Cells were transfected with 2500 ng of pHTN-TANGO1-CC2, 25 ng of pNLFN-cTAGE5-CC2, 3.75 µl of Lipofectamine 3000 and 5 µl of P3000 Reagent (Thermo Fisher Scientific). After 20 h, each transformation was re-plated in four wells of 96-well plates at a density of 1 × 10⁴ cells per well for duplicate control and experimental samples. PPIs were analyzed with NanoBRET^{™} Nano-Glo^{®} Detection System kit (Promega) following manufacturer's instructions. The corrected NanoBRET ratio was calculated according to the manufacturer's instructions. Each PPI combination was tested in duplicate wells (1 × 10⁴ cells per well) for all of the peptides and the peptide concentration ranges. Each experiment was carried out with duplicate wells.

### Imaging intracellular collagen in cell culture

Collagen visualization was carried out as before 9,21. Briefly, RDEB/FB/C7 cells grown in six-well plates were washed with PBS to remove the medium and replaced in fresh DMEM supplemented with 10% FCS. The cell permeable peptides (P2, P5) were then added at 10 µM final concentration for 20h, with ascorbate (250 µM ascorbic acid, 1 mM phospho-ascorbate) in full medium.

U2OS cells grown in petri dishes were washed to remove the medium and replaced in fresh medium. The cell permeable peptides (P2, P5) were then added at 10 µM final concentration for 4h, with ascorbate (250 µM ascorbic acid, 1mM phospho-ascorbate) in OptiMEM. Control has nothing added.

Cells were fixed and processed for immunofluorescence microscopy.

### Zebrafish experiments

### Zebrafish maintenance

For the peptide incubation experiments, AB wild-type zebrafish were used. zebrafish were maintained at Parc de Recerca Biomèdica de Barcelona (PRBB) according to the standard procedures of the aquatic facility. All the protocols were following the national and European guidelines and were approved by the Institutional Animal Care and Use Ethic Committee (PRBB-IACUEC). The principles of the 3Rs were applied in all zebrafish experiments.

The zebrafish wound healing experiments were approved by the local and federal animal care committees (City of Cologne: 8.87-50.10.31.08.129; LANUV Nordrhein-Westfalen: 81-02.04.2018.A097).

### Incubation

Zebrafish embryos were kept in E3 medium (5 mM NaCl, 0.17 mM KCI, 0.33 mM CaCl2, 0.33 M MgSO4) prior to the experiment. Zebrafish embryos were manually dechorionated between 2-4 cell stage to ensure that all eggs used in the experiment were viable. A total of 20 embryos per condition were immediately transferred to a 4-well plate with 5 embryos per well (Nunc delta, Thermo Fisher 176740) pre-filled with 500 µl of E3 medium with peptide inhibitors (2 µM or 4 µM of both P2 and P5). Control embryos were kept in E3 only, and medium changes were carried out at 1 dpf and 3 dpf for all treatment conditions. Embryos and larvae were continuously treated over the duration of the experiment and kept at 28°C. To avoid pigmentation in the zebrafish larvae, 0.1% PTU was added to the medium from 24 hpf. Embryos were monitored at sphere, shield, 75% epiboly, 1 dpf, 2dpf and 3 dpf for survival and development. At 3dpf, a representative group of larvae were imaged for phenotyping and length measurements. The experiment was carried out three times. For imaging of collagen fibers with second harmonic generation microscopy, larvae were fixed in 4% PFA at 4°C overnight, followed by a PBS wash.

### Wound healing in zebrafish

Adult zebrafish of the TL/Ekwill wildtype strain at an age 8 months were used for wounding experiments. Full-thickness wounds of adult fish with a diameter of ~2 mm were introduced with a Dermablate laser, as described previously 28. In short, adult fish were anesthetized in 0.13% Tricaine (w/v) and placed in a lateral position on Whatman paper soaked in system water. Full-thickness wounds were introduced onto the left flank directly anterior to anal and dorsal fins. An Erbium:YAG MCL29 Dermablate dermatology laser Asclepion, Jena, Germany) was set to a frequency of 1 Hz and 5 pulses with a strength of 500 mJ were applied.

For local treatment of wounds with peptides, wounds were microinjected both at 2 and at 3 days post wounding (dpw), using a glass needle with a tip diameter of less than 50 µm, a micromanipulator and a Pneumatic PicoPump (PV820, World Precision Instruments). Wounds were injected at five sites, an inner site directly in the centre of the wound and four outer sites approximately 0.3 mm from the edge of the wound and at maximal distances from each other, thus forming a pattern as the five dots on a game die. Per wound, a total of 80 nl of a 50 µM peptide stock solution in phosphate buffered saline (PBS), pH 7.4 (Sigma) was injected. Assuming a granulation tissue volume of ~1 mm3 (µl), as calculated from its dimensions in our histological sections, this should yield a final peptide concentration within the granulation tissue of ~4 µM. As control, plain PBS was injected. For histological analyses, wounded and injected fish were sacrificed at 4 dpw and fixed in 4% paraformaldehyde/ PBS overnight at room temperature, decalcified in 0.5 M EDTA (pH 7.4) at room temperature for 5 days, dehydrated in a graded series of alcohols, cleared in Roti-Histol (Carl Roth, Karlsruhe, Germany) and embedded in paraffin. Sections of 10 µm thickness were stained with hematoxylin and eosin using standard protocols. For quantification of granulation tissue sizes, consecutive sections of wounds were stained with hematoxylin and eosin. Sections comprising the center of the wound were selected, granulation tissue areas were measured using imaged and Student's t-test was used to calculate significance. For immunohistochemical analysis, consecutive paraffin sections after the hydration step were microwaved in a preheated 10 mM citrate buffer (pH 6.0, 0.05% Tween 20) for 15 min. The slides were then left to cool at room temperature for 20 min and washed with PBS-Tween 20 (0.1%) three times for 10 min each. Blocking (10% FBS + 1% DMSO in PBST) was done for 3h at room temperature. Anti-col1 (1:200, ab23730, Abcam, Cambridge, UK) primary antibody in blocking buffer were added to the slides for incubation overnight at 4°C. The following day, the slides were washed with PBST four times for 10 min each and goat anti-rabbit Cy3 (1:750, RRIDAB AB_2534029, cat. no. A10520) secondary antibody in the blocking buffer were added to the slides for incubation at room temperature for 90 min. The slides were then washed again with PBST four times for 10 min each and mounted with Mowiol including DAPI. The slides were stored at 4°C until further imaging. Images were captured on a Zeiss Apotome microscope and collagen-I stained area in the granulation tissue were measured using imaged and Student's t-test was used to calculate the significance.

The ECM production was analysed at 4 dpw since the collagen accumulation in the granulation tissue of zebrafish cutaneous wounds reaches to its maximum at this stage.

### TUNEL staining

TUNEL staining on paraffin sections of the same individuals, also used for the Col2 immunofluorescence analysis, was performed using the DeadEndTM Fluorometric TUNEL System (Promega, G3250) and following the manufacturers' protocol, except prolonging the Proteinase K (provided in the kit) treatment to 30 min and adding 2 PBS-TritonTM-X-100 0,1 % wash steps following the rTdT enzyme reaction. Specificity of the assay was confirmed via negative and positive (DNAse treatment) controls. Images were taken on a Zeiss Axio Imager Z.1 microscope with apotome function. Images were processed using the mpl-inferno LUT application of the imaged software (Version 1.54f). For quantification, 5 individual fish and 2 individual sections per fish were analyzed.

### Second Harmonic Generation (SHG) microscopy of Zebrafish fin collagen fibers

To visualize collagen fibers, the yolk of the fixed larvae was removed manually to improve imaging. PFA-fixed and de-yolked zebrafish were mounted in 1% low melting point agarose (Invitrogen 16520050) on 50 mm bottom-glass dishes (P50G-0-14-F, MatTek Corporation) sandwiched with a round cover glass (30 mm diameter #1, Menzel Gläser and sealed with nail polish, to allow imaging in forward and backward direction. The experiment was carried out three times and a total of three control and three peptide-treated fish were imaged using second harmonic generation microscopy. The optical set up consists of a confocal microscope (Eclipse Ti-U, Nikon) adapted to perform nonlinear microscopy. The system was pumped with a femtosecond-pulsed laser beam (Halite, Fluence) operating at a central wavelength of 1030 nm. The laser was transversal scanned with a pair of two galvanometric mirrors in tandem configuration (Cambridge Technology). The beam was expanded with a telescope arrangement to fill the microscope objective pupil and directed with a dichroic mirror (DMS1000R, Thorlabs) to a 20x NA 0.75 air objective (Plan Apo λ, Nikon) to focus the beam on the sample. The signal was detected in forward direction with a 25x NA 1.10 water objective (Apo LWD, Nikon). The SHG signal is collected with a photomultiplier tube (H7422-40, Hamamatsu) a using a dichroic mirror (FF665-Di01), an IR filter (FGB37-A, Thorlabs) and a bandpass filter centered at 515 nm (FF01-515/30-25, Semrock). The transmitted light from the femtosecond-pulsed laser was collected with a silicon photodiode for sample reference. Image acquisition was obtained with an in-house implemented LabVIEW-based software. Z-stacks of 512x512 pixel images were reconstructed with FIJI (ImageJ, version 1.50i).

We quantified the preferred orientation of the collagen fibers from Control and P2+5(4µM)-treated fishes using the Directionality plugin from FIJI (imaged, version 1.50i). Data normalization and plot was performed using GraphPad Prism version 9.3.1 for Windows, GraphPad Software.

### Fibroblasts from human donors

Skin biopsies were obtained from healthy individuals or patients with scleroderma (systemic sclerosis) after written consent and with approval of the local ethics committee. Primary dermal fibroblasts were cultured by explant outgrowth in DMEM (Gibco Invitrogen, 41965-039) with 100 U/ml penicillin, 100 µg/ml streptomycin (Sigma, P0781), 2 mM L-glutamine (Biozym, 882027) 50 µg/ml Na-ascorbate (Sigma-Aldrich, A4034) and 10% fetal calf serum (Gibco, 10270-106) in the moist atmosphere of a CO2 incubator. Cells were used in passage 2-5.

### Peptide uptake into primary skin fibroblasts

For visualization of peptide uptake, fibroblasts were seeded at 1.4×10⁴ into an 8 well chambered coverslip (Ibidi) and grown confluent overnight. To visualize the ER, cells were treated with CellLight^{™} ER-RFP (invitrogen) for24 h. Then, peptide solutions of serum-free medium containing either 40 µM CF-labeled P2 or P5, or both peptides combined were added. After 20 h, cell nuclei were stained with Hoechst 33342 nuclear dye (Thermo Fisher) for 10 min. Live fluorescence microscopy images were taken using the Keyence BZ-X800 microscope and afterwards, images were processed using imaged.

### Secretion assays in fibroblasts from human donors

To assess secretion, fibroblasts were seeded at high density (3×10⁵ per 6-well) and grown to confluence overnight, washed extensively and treated in serum-free medium with either P2 or P5 or the combination of both peptides or the scrambled peptides scrP2, scrP5 or the combination of them at the indicated concentrations for 20 h. For some experiments, fibroblasts were pretreated with TGFβ1 (10 ng/ml; R&D systems, 240-B) for 12 hours in the presence of 10 % fetal calf serum, washed extensively and serum-free media with TGFβ1 and peptides were added for 20 h.

To assay the duration of effect of the peptides, cells were treated without (Ctrl) or with 40 µM each of P2+P5 for20 h in the absence of serum, and supernatants were removed. Then, growth medium containing 10% serum but no peptides were supplied to control and peptide-treated cultures for 24, 48 or 72 h.

Equal volumes of supernatants were analyzed by SDS-PAGE and transferred to PVDF membranes (Immobilon, Merck, 0.45 µm, IPVH00010). This was possible because total protein concentrations in the corresponding lysates were comparable, as secured by BCA determination of total protein content (PierceTM BCA Protein Assay Kits (Thermo Scientific, 23225)) and Ponceau staining (Sigma-Aldrich, P7170) of membranes with proteins from the cell lysates. Primary antibodies used were: Goat anti-type I collagen (SouthernBiotech, 1310-01; 1:2000; reduced); rabbit anti-fibrillin1 (kind gift from G. Sengle, Cologne; 1:2000 unreduced); rabbit anti-fibronectin (abcam, ab23750; 1:500, unreduced); and rabbit anti-GAPDH (abcam, ab9485; 1:2500; reduced), followed by appropriate horseradish peroxidase-conjugated secondary antibodies and detection using enhanced chemiluminescence (Thermo Scientific 34087). Intracellular collagen I retention was analyzed in fibroblast lysates (20mM Tris-HCl pH 7.5, 1% SDS, 0.5% NP-40, 2 mM EDTA) and normalized to GAPDH (abcam, ab9485; 1:2500; reduced). Band intensities were measured using the Gel Analysis tool in imaged software.

Cytotoxic effects induced by peptide treatment were determined by lactate dehydrogenase (LDH) levels in fibroblast supernatants using Cytotoxicity Assay (Promega G1780) according to the manufacturer's instructions.

### Secretome analysis

Sample preparation. Supernatants from treated fibroblasts and controls were also used for proteomics analysis to determine the secretome. Supernatants were removed from the cultures, filtered and clarified through centrifugation. Protease inhibitor was added, and samples were frozen at -80°C until further processing. Proteins from supernatants were acetone precipitated and resuspended in 6 M urea/ 2 M thiourea. Disulfide reduction and alkylation were performed using 20 mM TCEP (Sigma-Aldrich) and 25 mM CAA (Merck) at RT for 1 h. Proteins were digested with endoproteinase Lys-C (Wako) (1:100 enzyme-to-substrate ratio) for 3 h at RT. Then, the urea concentration was diluted fourfold with 50 mM ABC buffer and digested with trypsin (Promega/Sigma) (1:100 enzyme-to-substrate ratio) overnight at RT. Digestion was stopped by adding formic acid to a final concentration of 1%. The samples were desalted by Stop-and-Go extraction using SDB-RPS StageTips.

LC-MS/MS analysis and data processing. Secretome analyses were performed using an Easy nLC 1000 ultra-high performance liquid chromatography (UHPLC) coupled to a QExactive Plus mass spectrometer (Thermo Fisher Science) with the same settings as described before. Acquired MS spectra were correlated to the mouse FASTA database using MaxQuant (V. 1.5.38) with its implemented Andromeda search engine. All parameters were set to default. N-terminal acetylation and methionine oxidation were set as variable modifications and cysteine carbamidomethylation was set as fixed modification. Statistical analyses and GO annotations were performed in Perseus (V. 1.6.2.3.58) and data were visualized using InstantClue 51. Significance cutoff was set to a FDR < 0.05

Data availability. The mass spectrometry proteomics data have been deposited to the ProteomeXchange Consortium via the PRIDE partner repository with the dataset identifier PXD041678.

### Statistical Analyses

Statistical analyses were done using GraphPad Prism version 6. Applied tests are indicated in individual figure legends. * = p < 0.05; ** p < 0.01; *** p < 0.001; **** p < 0.0001.

### Sequences

| SEQ ID NO: | Descript or | Sequence |
|---|---|---|
| 1 | P1 | MILSDEAIKYKDKIKTLEKNQEILDDTAKN |
| 2 | P1+CP | MILSDEAIKYKDKIKTLEKNQEILDDTAKNGLRKRLRKFRNK |
| 3 | P2 | LRQKVEILNELYQQKEMALQKKLSQEEYER |
| 4 | P2+CP | LRQKVEILNELYQQKEMALQKKLSQEEYERGLRKRLRKFRNK |
| 5 | P3 | QEREHRLSAADEKAVSAAEEVKTYKRRIEE |
| 6 | P3+CP | QEREHRLSAADEKAVSAAEEVKTYKRRIEEGLRKRLRKFRNK |
| 7 | P4 | LQQKLKVMTELYQENEMKLHRKLTVEENYR |
| 8 | P4+CP | LQQKLKVMTELYQENEMKLHRKLTVEENYRGLRKRLRKFRNK |
| 9 | P5 | LEKEEKLSKVDEKISHATEELETYRKRAKDL |
| 10 | P5+CP | LEKEEKLSKVDEKISHATEELETYRKRAKDLGLRKRLRKFRNK |
| 11 | scrP2 | VEEAKLYQRQSRMENLLEKEKQELLQKQIYGLRKRLRKFRNK |
| 12 | scrP5 | KLRKHELERLKSELEEIEATETKDYVADKKSGLRKRLRKFRNK |
| 13 | Cell penetrat ing peptide (P6) | GLRKRLRKFRNK |
| 14 | TANGO1-Long | MAAAPGLLVWLLVLRLPWRVPGQLDPSTGRRFSEHKLCADDECSMLMYRGEALE DFTGPDCRFVNFKKGDPVYVYYKLARGWPEVWAGSVGRTFGYFPKDLIQVVHEY |
| | | TKEELQVPTDETDFVCFDGGRDDFHNYNVEELLGFLELYNSAATDSEKAVEKTL QDMEKNPELSKEREPEPEPEPVEANSEESDSVFSENTEDLQEQFTTQKHHSHANSQ ANHAQGEQASFESFEEMLQDKLKVPESENNKTSNSSQVSNEQDKIDAYKLLKKE MTLDLKTKFGSTADALVSDDETTRLVTSLEDDFDEELDTEYYAVGKEDEENQED FDELPLLTFTDGEDMKTPAKSGVEKYPTDKEQNSNEEDKVQLTVPPGIKNDDKN ILTTWGDTIFSIVTGGEETRDTMDLESSSSEEEKEDDDDALVPDSKQGKPQSAT DYSDPDNVDDGLFIVDIPKTNNDKEVNAEHHIKGKGRGVQESKRGLVQDKTELE DENQEGMTVHSSVHSNNLNSMPAAEKGKDTLKSAYDDTENDLKGAAIHISKGML HEEKPGEQILEGGSESESAQKAAGNQMNDRKIQQESLGSAPLMGDDHPNASRDS VEGDALVNGAKLHTLSVEHQREELKEELVLKTQNQPRFSSPDEIDLPRELEDEV PILGRNLPWQQERDVAATASKQMSEKIRLSEGEAKEDSLDEEFFHHKAMQGTEV GQTDQTDSTGGPAFLSKVEEDDYPSEELLEDENAINAKRSKEKNPGNQGRQFDV NLQVPDRAVLGTIHPDPEIEESKQETSMILDSEKTSETAAKGVNTGGREPNTMV EKERPLADKKAQRPFERSDFSDSIKIQTPELGEVFQNKDSDYLKNDNPEEHLKT SGLAGEPEGELSKEDHENTEKYMGTESQGSAAAEPEDDSFHWTPHTSVEPGHSD KREDLLIISSFFKEQQSLQRFQKYFNVHELEALLQEMSSKLKSAQQESLPYNME KVLDKVFRASESQILSIAEKMLDTRVAENRDLGMNENNIFEEAAVLDDIQDLIY FVRYKHSTAEETATLVMAPPLEEGLGGAMEEMQPLHEDNFSREKTAELNVQVPE EPTHLDQRVIGDTHASEVSQKPNTEKDLDPGPVTTEDTPMDAIDANKQPETAAE EPASVTPLENAILLIYSFMFYLTKSLVATLPDDVQPGPDFYGLPWKPVFITAFL GIASFAIFLWRTVLVVKDRVYQVTEQQISEKLKTIMKENTELVQKLSNYEQKIK ESKKHVQETRKQNMILSDEAIKYKDKIKTLEKNQEILDDTAKNLRVMLESEREQ NVKNQDLISENKKSIEKLKDVISMNASEFSEVQIALNEAKLSEEKVKSECHRVQ EENARLKKKKEQLQQEIEDWSKLHAELSEQIKSFEKSQKDLEVALTHKDDNINA LTNCITQLNLLECESESEGQNKGGNDSDELANGEVGGDRNEKMKNQIKQMMDVS RTQTAISVVEEDLKLLQLKLRASVSTKCNLEDQVKKLEDDRNSLQAAKAGLEDE CKTLRQKVEILNELYQQKEMALQKKLSQEEYERQEREHRLSAADEKAVSAAEEV KTYKRRIEEMEDELQKTERSFKNQIATHEKKAHENWLKARAAERAIAEEKREAA NLRHKLLELTQKMAMLQEEPVIVKPMPGKPNTQNPPRRGPLSQNGSFGPSPVSG GECSPPLTVEPPVRPLSATLNRRDMPRSEFGSVDGPLPHPRWSAEASGKPSPSD PGSGTATMMNSSSRGSSPTRVLDEGKVNMAPKGPPPFPGVPLMSTPMGGPVPPP IRYGPPPQLCGPFGPRPLPPPFGPGMRPPLGLREFAPGVPPGRRDLPLHPRGFL PGHAPFRPLGSLGPREYFIPGTRLPPPTHGPQEYPPPPAVRDLLPSGSRDEPPP ASQSTSQDCSQALKQSP |
| 15 | TANGO1 SH3-like domain | MLMYRGEALEDFTGPDCRFVNFKKGDPVYVYYKLARGWPEVWAGSVGRTFGYFP KDLIQVVHE |
| 16 | TANGO1 Lumenal coiled coil (CCL) | GVQESKRGLVQDKTELEDENQEGMTVHSSVHSNNLNSMPAAEKGKDTLKSAYDD TENDLKGAAIHISKGMLHEEKPGEQILEGGSESESAQ |
| 17 | TANGO1 Cytoplas mic coiled coil (CC1) | QQISEKLKTIMKENTELVQKLSNYEQKIKESKKHVQETRKQNMILSDEAIKYKD KIKTLEKNQEILDDTAKNLRVMLESEREQNVKNQDLISENKKSIEKLKDVISMN ASEFSEVQIALNEAKLSEEKVKSECHRVQEENARLKKKKEQLQQEIEDWSKLHA ELSEQIKSFEKSQKDLEVAL |
| 18 | TANGO1 Cytoplas mic coiled coil (CC2) | NLEDQVKKLEDDRNSLQAAKAGLEDECKTLRQKVEILNELYQQKEMALQKKLSQ EEYERQEREHRLSAADEKAVSAAEEVKTYKRRIEEMEDELQKTERSFKNQIATH EKKAHENWLKARAAERAIAEEKREAANLRHKLLELTQKMAMLQEEP |
| 19 | TANGO1 Proline rich domain (PRD) | SRGSSPTRVLDEGKVNMAPKGPPPFPGVPLMSTPMGGPVPPPIRYGPPPQLCGP FGPRPLPPPFGPGMRPPLGLREFAPGVPPGRRDLPLHPRGFLPGHAPFRPLGSL GPREYFIPGTRLPPPTHGPQEYPPPPAVRDLLPSGSRDEPPPASQSTSQDCSQA LKQSP |
| 20 | TANGO1-Short | MDSVPATVPSIAATPGDPELVGPLSVLYAAFIAKLLELVATLPDDVQPGPDFYG LPWKPVFITAFLGIASFAIFLWRTVLVVKDRVYQVTEQQISEKLKTIMKENTEL VQKLSNYEQKIKESKKHVQETRKQNMILSDEAIKYKDKIKTLEKNQEILDDTAK NLRVMLESEREQNVKNQDLISENKKSIEKLKDVISMNASEFSEVQIALNEAKLS EEKVKSECHRVQEENARLKKKKEQLQQEIEDWSKLHAELSEQIKSFEKSQKDLE VALTHKDDNINALTNCITQLNLLECESESEGQNKGGNDSDELANGEVGGDRNEK MKNQIKQMMDVSRTQTAISVVEEDLKLLQLKLRASVSTKCNLEDQVKKLEDDRN SLQAAKAGLEDECKTLRQKVEILNELYQQKEMALQKKLSQEEYERQEREHRLSA ADEKAVSAAEEVKTYKRRIEEMEDELQKTERSFKNQIATHEKKAHENWLKARAA ERAIAEEKREAANLRHKLLELTQKMAMLQEEPVIVKPMPGKPNTQNPPRRGPLS QNGSFGPSPVSGGECSPPLTVEPPVRPLSATLNRRDMPRSEFGSVDGPLPHPRW SAEASGKPSPSDPGSGTATMMNSSSRGSSPTRVLDEGKVNMAPKGPPPFPGVPL MSTPMGGPVPPPIRYGPPPQLCGPFGPRPLPPPFGPGMRPPLGLREFAPGVPPG RRDLPLHPRGFLPGHAPFRPLGSLGPREYFIPGTRLPPPTHGPQEYPPPPAVRD LLPSGSRDEPPPASQSTSQDCSQALKQSP |
| 21 | cTAGE5 | MEEPGVTPQPYLGLLLEELRRVVAALPEGMRPDSNLYGFPWELVICAAVVGFFA VLFFLWRSFRSVRSRLYVGREKKLALMLSGLIEEKSKLLEKFSLVQKEYEGYEV ESSLKDASFEKEATEAQSLEATCEKLNRSNSELEDEILCLEKELKEEKSKHSEQ DELMADISKRIQSLEDESKSLKSQVAEAKMTFKIFQMNEERLKIAIKDALNENS QLQESQKQLLQEAEVWKEQVSELNKQKVTFEDSKVHAEQVLNDKESHIKTLTER LLKMKDWAAMLGEDITDDDNLELEMNSESENGAYLDNPPKGALKKLIHAAKLNA SLKTLEGERNQIYIQLSEVDKTKEELTEHIKNLQTEQASLQSENTHFENENQKL QQKLKVMTELYQENEMKLHRKLTVEENYRLEKEEKLSKVDEKISHATEELETYR KRAKDLEEELERTIHSYQGQIISHEKKAHDNWLAARNAERNLNDLRKENAHNRQ KLTETELKFELLEKDPYALDVPNTAFGREHSPYGPSPLGWPSSETRAFLSPPTL LEGPLRLSPLLPGGGGRGSRGPGNPLDHQITNERGESSCDRLTDPHRAPSDTGS LSPPWDQDRRMMFPPPGQSYPDSALPPQRQDRFCSNSGRLSGPAELRSFNMPSL DKMDGSMPSEMESSRNDTKDDLGNLNVPDSSLPAENEATGPGFVPPPLAPIRGP LFPVDARGPFLRRGPPFPPPPPGAMFGASRDYFPPGDFPGPPPAPFAMRNVYPP RGFPPYLPPRPGFFPPPPHSEGRSEFPSGLIPPSNEPATEHPEPQQET |
| 22 | cTAGE5 CC1 | FEKEATEAQSLEATCEKLNRSNSELEDEILCLEKELKEEKSKHSEQDELMADIS KRIQSLEDESKSLKSQVAEAKMTFKIFQMNEERLKIAIKDALNENSQLQESQKQ LLQEAEVWKEQVSELNKQ |
| 23 | cTAGE5 CC2 | LSEVDKTKEELTEHIKNLQTEQASLQSENTHFENENQKLQQKLKVMTELYQENE MKLHRKLTVEENYRLEKEEKLSKVDEKISHATEELETYRKRAKDLEEELERTIH SYQGQIISHEKKAHDNWLAARNAERNLNDLRKENAHNRQKLTETELK |
| 24 | cTAGE5 Proline rich domain (PRD) | MDGSMPSEMESSRNDTKDDLGNLNVPDSSLPAENEATGPGFVPPPLAPIRGPLF PVDARGPFLRRGPPFPPPPPGAMFGASRDYFPPGDFPGPPPAPFAMRNVYPPRG FPPYLPPRPGFFPPPPHSEGRSEFPSGLIPPSNEPATEHPEPQQET |
| 25 | TALI | MAKFGVHRILLLAISLTKCLESTKLLADLKKCGDLECEALINRVSAMRDYRGPD CRYLNFTKGEEISVYVKLAGEREDLWAGSKGKEFGYFPRDAVQIEEVFISEEIQ MSTKESDFLCLLGVSYTFDNEDSELNGDYGENIYPYEEDKDEKSSIYESDFQIE PGFYATYESTLFEDQVPALEAPEDIGSTSESKDWEEVVVESMEQDRIPEVHVPP SSAVSGVKEWFGLGGEQAEEKAFESVIEPVQESSFRSRKIAVEDENDLEELNNG EPQTEHQQESESEIDSVPKTQSELASESEHIPKPQSTGWFGGGFTSYLGFGDED TGLELIAEESNPPLQDFPNSISSDKEATVPCTEILTEKKDTITNDSLSLKPSWF DFGFAILGFAYAKEDKIMLDDRKNEEDGGADEHEHPLTSELDPEKEQEIETIKI IETEDQIDKKPVSEKTDESDTIPYLKKFLYNFDNPWNFQNIPKETELPFPKQIL DQNNVIENEETGEFSIDNYPTDNTKVMIFKSSYSLSDMVSNIELPTRIHEEVYF EPSSSKDSDENSKPSVDTEGPALVEIDRSVENTLLNSQMVSTDNSLSSQNYISQ KEDASEFQILKYLFQIDVYDFMNSAFSPIVILTERVVAALPEGMRPDSNLYGFP WELVICAAVVGFFAVLFFLWRSFRSVRSRLYVGREKKLALMLSGLIEEKSKLLE KFSLVQKEYEGYEVESSLKDASFEKEATEAQSLEATCEKLNRSNSELEDEILCL EKELKEEKSKHSEQDELMADISKRIQSLEDESKSLKSQVAEAKMTFKIFQMNEE RLKIAIKDALNENSQLQESQKQLLQEAEVWKEQVSELNKQKVTFEDSKVHAEQV LNDKESHIKTLTERLLKMKDWAAMLGEDITDDDNLELEMNSESENGAYLDNPPK GALKKLIHAAKLNASLKTLEGERNQIYIQLSEVDKTKEELTEHIKNLQTEQASL QSENTHFENENQKLQQKLKVMTELYQENEMKLHRKLTVEENYRLEKEEKLSKVD EKISHATEELETYRKRAKDLEEELERTIHSYQGQIISHEKKAHDNWLAARNAER NLNDLRKENAHNRQKLTETELKFELLEKDPYALDVPNTAFGREHSPYGPSPLGW PSSETRAFLSPPTLLEGPLRLSPLLPGGGGRGSRGPGNPLDHQITNERGESSCD RLTDPHRAPSDTGSLSPPWDQDRRMMFPPPGQSYPDSALPPQRQDRFCSNSGRL SGPAELRSFNMPSLDKMDGSMPSEMESSRNDTKDDLGNLNVPDSSLPAENEATG PGFVPPPLAPIRGPLFPVDARGPFLRRGPPFPPPPPGAMFGASRDYFPPGDFPG PPPAPFAMRNVYPPRGFPPYLPPRPGFFPPPPHSEGRSEFPSGLIPPSNEPATE HPEPQQET |
| 26 | TALI SH3-like domain | ALINRVSAMRDYRGPDCRYLNFTKGEEISVYVKLAGEREDLWAGSKGKEFGYFP RDAVQIEEV |
| 27 | NBAS | MAAPESGPALSPGTAEGEEETILYDLLVNTEWPPETEVQPRGNQKHGASFIITK AIRDRLLFLRQYIWYSPAPFLLPDGLVRLVNKQINWHLVLASNGKLLAAVQDQC VEIRSAKDDFTSIIGKCQVPKDPKPQWRRVAWSYDCTLLAYAESTGTVRVFDLM GSELFVISPASSFIGDLSYAIAGLIFLEYKASAQWSAELLVINYRGELRSYLVS VGTNQSYQESHCFSFSSHYPHGINTAIYHPGHRLLLVGGCETAEVGMSKASSCG LSAWRVLSGSPYYKQVTNGGDGVTAVPKTLGLLRMLSVKFYSRQGQEQDGIFKM SLSPDGMLLAAIHFSGKLSIWAIPSLKQQGEWGQNEQPGYDDLNPDWRLSTEKR KKIKDKESFYPLIDVNWWADSAVTLARCSGALTVSSVKTLKNLLGKSCEWFEPS PQVTATHDGGFLSLECEIKLAPKRSRLETRAGEEDEGEEDSDSDYEISAKARYF GYIKQGLYLVTEMERFAPPRKRPRTITKNYRLVSLRSTTPEELYQRKIESEEYE EALSLAHTYGLDTDLVYQRQWRKSAVNVASIQNYLSKIKKRSWVLHECLERVPE NVDAAKELLQYGLKGTDLEALLAIGKGADDGRFTLPGEIDIDSISYEELSPPDE EPAKNKKEKELKKRQELLKLVNFSKLTLEQKELCRCRRKLLTYLDRLATYEEIL GVPHASEQRYDAEFFKKFRNQNIVLSARTYAQESNVQALEILFTYHGSDLLPHR LAILSNFPETTSPHEYSVLLPEACFNGDSLMIIPWHEHKHRAKDWCEELACRMV VEPNLQDESEFLYAAQPELLRFRMTQLTVEKVMDWYQTRAEEIEHYARQVDCAL SLIRLGMERNIPGLLVLCDNLVTLETLVYEARCDVTLTLKELQQMKDIEKLRLL MNSCSEDKYVTSAYQWMVPFLHRCEKQSPGVANELLKEYLVTLAKGDLKFPLKI |
| | | FQHSKPDLQQKIIPDQDQLMAIALECIYTCERNDQLCLCYDLLECLPERGYGDK TEATTKLHDMVDQLEQILSVSELLEKHGLEKPISFVKNTQSSSEEARKLMVRLT RHTGRKQPPVSESHWRTLLQDMLTMQQNVYTCLDSDACYEIFTESLLCSSRLEN IHLAGQMMHCSACSENPPAGIAHKGKPHYRVSYEKSIDLVLAASREYFNSSTNL TDSCMDLARCCLQLITDRPPAIQEELDLIQAVGCLEEFGVKILPLQVRLCPDRI SLIKECISQSPTCYKQSTKLLGLAELLRVAGENPEERRGQVLILLVEQALRFHD YKAASMHCQELMATGYPKSWDVCSQLGQSEGYQDLATRQELMAFALTHCPPSSI ELLLAASSSLQTEILYQRVNFQIHHEGGENISASPLTSKAVQEDEVGVPGSNSA DLLRWTTATTMKVLSNTTTTTKAVLQAVSDGQWWKKSLTYLRPLQGQKCGGAYQ IGTTANEDLEKQGCHPFYESVISNPFVAESEGTYDTYQHVPVESFAEVLLRTGK LAEAKNKGEVFPTTEVLLQLASEALPNDMTLALAYLLALPQVLDANRCFEKQSP SALSLQLAAYYYSLQIYARLAPCFRDKCHPLYRADPKELIKMVTRHVTRHEHEA WPEDLISLTKQLHCYNERLLDFTQAQILQGLRKGVDVQRFTADDQYKRETILGL AETLEESVYSIAISLAQRYSVSRWEVFMTHLEFLFTDSGLSTLEIENRAQDLHL FETLKTDPEAFHQHMVKYIYPTIGGFDHERLQYYFTLLENCGCADLGNCAIKPE THIRLLKKFKVVASGLNYKKLTDENMSPLEALEPVLSSQNILSISKLVPKIPEK DGQMLSPSSLYTIWLQKLFWTGDPHLIKQVPGSSPEWLHAYDVCMKYFDRLHPG DLITVVDAVTFSPKAVTKLSVEARKEMTRKAIKTVKHFIEKPRKRNSEDEAQEA KDSKVTYADTLNHLEKSLAHLETLSHSFILSLKNSEQETLQKYSHLYDLSRSEK EKLHDEAVAICLDGQPLAMIQQLLEVAVGPLDISPKDIVQSAIMKIISALSGGS ADLGGPRDPLKVLEGVVAAVHASVDKGEELVSPEDLLEWLRPFCADDAWPVRPR IHVLQILGQSFHLTEEDSKLLVFFRTEAILKASWPQRQVDIADIENEENRYCLF MELLESSHHEAEFQHLVLLLQAWPPMKSEYVITNNPWVRLATVMLTRCTMENKE GLGNEVLKMCRSLYNTKQMLPAEGVKELCLLLLNQSLLLPSLKLLLESRDEHLH EMALEQITAVTTVNDSNCDQELLSLLLDAKLLVKCVSTPFYPRIVDHLLASLQQ GRWDAEELGRHLREAGHEAEAGSLLLAVRGTHQAFRTFSTALRAAQHWV |
| 28 | RINT1 | MLPAGEIGASPAAPCCSESGDERKNLEEKSDINVTVLIGSKQVSEGTDNGDLPS YVSAFIEKEVGNDLKSLKKLDKLIEQRTVSKMQLEEQVLTISSEIPKRIRSALK NAEESKQFLNQFLEQETHLFSAINSHLLTAQPWMDDLGTMISQIEEIERHLAYL KWISQIEELSDNIQQYLMTNNVPEAASTLVSMAELDIKLQESSCTHLLGFMRAT VKFWHKILKDKLTSDFEEILAQLHWPFIAPPQSQTVGLSRPASAPEIYSYLETL FCQLLKLQTSDELLTEPKQLPEKYSLPASPSVILPIQVMLTPLQKRFRYHFRGN RQTNVLSKPEWYLAQVLMWIGNHTEFLDEKIQPILDKVGSLVNARLEFSRGLMM LVLEKLATDIPCLLYDDNLFCHLVDEVLLFERELHSVHGYPGTFASCMHILSEE TCFQRWLTVERKFALQKMDSMLSSEAAWVSQYKDITDVDEMKVPDCAETFMTLL LVITDRYKNLPTASRKLQFLELQKDLVDDFRIRLTQVMKEETRASLGFRYCAIL NAVNYISTVLADWADNVFFLQLQQAALEVFAENNTLSKLQLGQLASMESSVFDD MINLLERLKHDMLTRQVDHVFREVKDAAKLYKKERWLSLPSQSEQAVMSLSSSA CPLLLTLRDHLLQLEQQLCFSLFKIFWQMLVEKLDVYIYQEIILANHFNEGGAA QLQFDMTRNLFPLFSHYCKRPENYFKHIKEACIVLNLNVGSALLLKDVLQSASG QLPATAALNEVGIYKLAQQDVEILLNLRTNWPNTGK |
| 29 | ZW10 | MASFVTEVLAHSGRLEKEDLGTRISRLTRRVEEIKGEVCNMISKKYSEFLPSMQ SAQGLITQVDKLSEDIDLLKSRIESEVRRDLHVSTGEFTDLKQQLERDSVVLSL LKQLQEFSTAIEEYNCALTEKKYVTGAQRLEEAQKCLKLLKSRKCFDLKILKSL SMELTIQKQNILYHLGEEWQKLIVWKFPPSKDTSSLESYLQTELHLYTEQSHKE EKTPMPPISSVLLAFSVLGELHSKLKSFGQMLLKYILRPLASCPSLHAVIESQP NIVIIRFESIMTNLEYPSPSEVFTKIRLVLEVLQKQLLDLPLDTDLENEKTSTV PLAEMLGDMIWEDLSECLIKNCLVYSIPTNSSKLQQYEEIIQSTEEFENALKEM RFLKGDTTDLLKYARNINSHFANKKCQDVIVAARNLMTSEIHNTVKIIPDSKIN VPELPTPDEDNKLEVQKVSNTQYHEVMNLEPENTLDQHSFSLPTCRISESVKKL |
| | | |
| 30 | Tat (48-60) | GRKKRRQRRRPPQ |
| 31 | Penetrat in | RQIKIWFQNRRMKWKK |
| 32 | VP22 | RPRAPARSASRPRRPVE |
| 33 | R9 | RRRRRRRRR |
| 34 | sC18 | GLRKRLRKFRNKIKEK |
| 35 | MAP | KLALKLALKALKAALKLA |
| 36 | Pept1 | PLILLRLLRGQF |
| 37 | Halo-T1-CC2 | MAEIGTGFPFDPHYVEVLGERMHYVDVGPRDGTPVLFLHGNPTSSYVWRNIIPH VAPTHRCIAPDLIGMGKSDKPDLGYFFDDHVRFMDAFIEALGLEEVVLVIHDWG SALGFHWAKRNPERVKGIAFMEFIRPIPTWDEWPEFARETFQAFRTTDVGRKLI IDQNVFIEGTLPMGVVRPLTEVEMDHYREPFLNPVDREPLWRFPNELPIAGEPA NIVALVEEYMDWLHQSPVPKLLFWGTPGVLIPPAEAARLAKSLPNCKAVDIGPG LNLLQEDNPDLIGSEIARWLSTLEISGEPTTEDLYFQSDNAIASELRQKVEILN ELYQQKEMALQKKLSQEEYERQEREHRLSAADEKAVSAAEEVKTYKRRIEEMED ELQKTERSFKNQIATHEKKAHENWLKARAAERAIAEEKREAANLRHKLLELTQK MAMLQEEP |
| 38 | NanoLuc-cTAGE5-CC2 | MVFTLEDFVGDWRQTAGYNLDQVLEQGGVSSLFQNLGVSVTPIQRIVLSGENGL KIDIHVIIPYEGLSGDQMGQIEKIFKVVYPVDDHHFKVILHYGTLVIDGVTPNM IDYFGRPYEGIAVFDGKKITVTGTLWNGNKIIDERLINPDGSLLFRVTINGVTG WRLCERILAGSSGAIASEKLQQKLKVMTELYQENEMKLHRKLTVEENYRLEKEE KLSKVDEKISHATEELETYRKRAKDLEEELERTIHSYQGQIISHEKKAHDNWLA ARNAERNLNDLRKENAHNRQKLTETELKFELLEKDPY |

### Clauses

1. An inhibitor of TANGO1 activity, wherein the inhibitor is comprised of one or more inhibitors selected from the group consisting of a polypeptide, a small molecule, a nucleic acid, an antibody, and an antibody fragment.
2. An inhibitor of TANGO1 heterodimerisation, wherein the inhibitor is comprised of one or more inhibitors selected from the group consisting of a polypeptide, a small molecule, a nucleic acid, an antibody, and an antibody fragment.
3. The inhibitor of clause 1 or 2, wherein the inhibitor binds to one or more target proteins selected from the group consisting of TANGO1, TALI, and cTAGE5.
4. The inhibitor of any one of the preceding clauses, wherein the inhibitor prevents heterodimerisation of TANGO1 with TALI and/or cTAGE5.
5. The inhibitor of any one of the preceding clauses, wherein the TANGO1 is the short- or the long-form isomer of TANGO1.
6. The inhibitor of any one of the preceding clauses, wherein the TANGO1 is the short-isomer of TANGO1 having the sequence of SEQ ID NO: 20.
7. The inhibitor of any one of the preceding clauses, wherein the TANGO1 is the long-isomer of TANGO1 having the sequence of SEQ ID NO: 14.
8. The inhibitor of any one of the preceding clauses, wherein the inhibitor binds to a cytoplasmic portion of TANGO1, TALI, and/or cTAGE5.
9. The inhibitor of any one of the preceding clauses, wherein the inhibitor binds to a region within one or more cytoplasmic coiled-coil domains of the target protein.
10. The inhibitor of any one of the preceding clauses, wherein the inhibitor binds to a region within SEQ ID NO: 17, SEQ ID NO: 18, SEQ ID NO: 22, and/or SEQ ID NO: 23.
11. The inhibitor of any one of the preceding clauses, wherein the one or more inhibitors bind to a region within SEQ ID NO: 17, SEQ ID NO: 18, and/or SEQ ID NO: 23.
12. The inhibitor of any one of the preceding clauses, wherein the one or more inhibitors bind to a region within SEQ ID NO: 18, and/or SEQ ID NO: 23.
13. The inhibitor of any one of the preceding clauses, wherein the inhibitor inhibits TANGO1-cTAGE5 heterodimerisation.
14. The inhibitor of any one of the preceding clauses, wherein the inhibitor inhibits TANGO1-TALI heterodimerisation.
15. The inhibitor of any of the preceding clauses, wherein inhibition of TANGO1 heterodimerisation results in loss of function of the TANGO1 protein and/or a reduction in collagen export and/or ECM proteins from the endoplasmic reticulum.
16. The inhibitor of any one of the preceding clauses, wherein the inhibitor comprises one or more polypeptides having at least 50%, at least 60%, at least 70%, at least 80%, at least 90%, at least 95%, at least 98%, or 100% sequence identity to the sequences selected from the group consisting of SEQ ID NO: 1 to SEQ ID NO: 10.
17. The inhibitor of any one of the preceding clauses, wherein the inhibitor comprises a polypeptide having at least 50%, at least 60%, at least 70%, at least 80%, at least 90%, at least 95%, at least 98%, or 100% sequence identity to SEQ ID NOs: 3 to 6.
18. The inhibitor of any one of clauses 1 to 17, wherein the inhibitor binds to a region within SEQ ID NO: 23.
19. The inhibitor of clause 18, wherein the inhibitor binds to a region within residues 59 to 109 of SEQ ID NO: 23.
20. The inhibitor of clause 18, wherein the inhibitor binds to a region within residues 64 to 104 of SEQ ID NO: 23.
21. The inhibitor of clause 18, wherein the inhibitor binds to a region within residues 67 to 101 of SEQ ID NO: 23.
22. The inhibitor of clause 18, wherein the inhibitor binds to a region within residues 68 to 100 of SEQ ID NO: 23.
23. The inhibitor of clause 18, wherein the inhibitor binds to a region within residues 69 to 99 of SEQ ID NO: 23.
24. The inhibitor of clause 18, wherein the inhibitor binds within the region starting from residue 59, 64, 67, 68, or 69, and ending at residue 109, 104, 101, 100, or 99, of SEQ ID NO: 23.
25. The inhibitor of clause 18, wherein the inhibitor binds to a region within residues 29 to 78 of SEQ ID NO: 23.
26. The inhibitor of clause 18, wherein the inhibitor binds to a region within residues 34 to 73 of SEQ ID NO: 23.
27. The inhibitor of clause 18, wherein the inhibitor binds to a region within residues 37 to 70 of SEQ ID NO: 23.
28. The inhibitor of clause 18, wherein the inhibitor binds to a region within residues 38 to 69 of SEQ ID NO: 23.
29. The inhibitor of clause 18, wherein the inhibitor binds to a region within residues 39 to 68 of SEQ ID NO: 23.
30. The inhibitor of clause 18, wherein the inhibitor binds within the region starting from residue 29, 34, 37, 38, or 39, and ending at residue 78, 73, 70, 69, or 68, of SEQ ID NO: 23.
31. The inhibitor of any one of clauses 18to 30, wherein the inhibitor binds to a region at least 5, at least 10, at least 15, at least 20, at least 25, at least 30, at least 35, at least 40, at least 45, at least 50, at least 55, or at least 60 residues in length.
32. The inhibitor of clause 18, wherein the inhibitor binds to a sequence according to SEQ ID NO: 7 or SEQ ID NO: 9.
33. The inhibitor of any one of the preceding clauses, wherein the inhibitor comprises a polypeptide having at least 50%, at least 60%, at least 70%, at least 80%, at least 90%, at least 95%, at least 98%, or 100% sequence identity to SEQ ID NOs: 7 to 10.
34. The inhibitor of any one of clauses 1 to 17, wherein the inhibitor binds to a region within SEQ ID NO: 18.
35. The inhibitor of clause 34, wherein the inhibitor binds to a region within residues 20 to 69 of SEQ ID NO: 18.
36. The inhibitor of clause 34, wherein the inhibitor binds to a region within residues 25 to 64 of SEQ ID NO: 18.
37. The inhibitor of clause 34, wherein the inhibitor binds to a region within residues 28 to 61 of SEQ ID NO: 18.
38. The inhibitor of clause 34, wherein the inhibitor binds to a region within residues 29 to 60 of SEQ ID NO: 18.
39. The inhibitor of clause 34, wherein the inhibitor binds to a region within residues 30 to 59 of SEQ ID NO: 18.
40. The inhibitor of clause 34, wherein the inhibitor binds within the region starting from residue 20, 25, 28, 29, or 30, and ending at residue 69, 64, 61, 60, or 59, of SEQ ID NO: 18.
41. The inhibitor of clause 34, wherein the inhibitor binds to a region within residues 50 to 99 of SEQ ID NO: 18.
42. The inhibitor of clause 34, wherein the inhibitor binds to a region within residues 55 to 94 of SEQ ID NO: 18.
43. The inhibitor of clause 34, wherein the inhibitor binds to a region within residues 58 to 91 of SEQ ID NO: 18.
44. The inhibitor of clause 34, wherein the inhibitor binds to a region within residues 59 to 90 of SEQ ID NO: 18.
45. The inhibitor of clause 34, wherein the inhibitor binds to a region within residues 60 to 89 of SEQ ID NO: 18.
46. The inhibitor of clause 34, wherein the inhibitor binds within the region starting from residue 50, 55, 58, 59, or 60, and ending at residue 99, 94, 91, 90, or 89, of SEQ ID NO: 18.
47. The inhibitor of any one of clauses 30 to 42, wherein the inhibitor binds to a region at least 5, at least 10, at least 15, at least 20, at least 25, at least 30, at least 35, at least 40, at least 45, at least 50, at least 55, or at least 60 residues in length.
48. The inhibitor of clause 34, wherein the inhibitor binds to a sequence according to SEQ ID NO: 3 or SEQ ID NO: 5.
49. The inhibitor of any preceding clause, wherein the inhibitor further comprises one or more signal sequences.
50. The inhibitor of clause 49, wherein the one or more signal sequences comprise a polypeptide selected from the group consisting of SEQ ID NO: 13, SEQ ID NO: 30, SEQ ID NO: 31, SEQ ID NO: 32, SEQ ID NO: 33, SEQ ID NO: 34, SEQ ID NO: 35, and SEQ ID NO: 36.
51. The inhibitor of clause 49, wherein the one or more signal sequences encode one or more polypeptides selected from the group consisting of SEQ ID NO: 13, SEQ ID NO: 30, SEQ ID NO: 31, SEQ ID NO: 32, SEQ ID NO: 33, SEQ ID NO: 34, SEQ ID NO: 35, and SEQ ID NO: 36.
52. The inhibitor of clause 50 or 51, wherein the signal sequence is appended to the polypeptide.
53. The inhibitor of clause 52, wherein the signal sequence is appended to the N' terminus of the polypeptide.
54. The inhibitor of clause 52, wherein the signal sequence is appended to the C' terminus of the polypeptide.
54a. The inhibitor of any one of the preceding clauses, wherein the nucleic acid is RNA.
55. The inhibitor of clause 54a, wherein the RNA is selected from the group consisting of shRNA, siRNA, miRNA, antisense oligonucleotides, RNA aptamers, modified RNA, native RNA, and combinations thereof.
56. The antisense oligonucleotide of clause 55, wherein the antisense oligonucleotide is
   (a) between 7 and 15 nucleotides long, and wherein the oligonucleotide is a locked nucleic acid;
   (b) between 18 and 25 nucleotides long, and wherein the oligonucleotide is a 2'-O-methyl phosphorothioate ribonucleic acid; or
   (c) between 18 and 25 nucleotides long, and wherein the oligonucleotide is a 2'-O-methoxyethyl-modified phosphorothioate ribonucleic acid.
57. The antisense oligonucleotide of clause 55 or 56, wherein at least one cytosine residue within the oligonucleotide is methylated.
58. The inhibitor of any one of the preceding clauses, wherein the antibody is selected from the group consisting of a monoclonal, polyclonal, bispecific, and chimeric antibody.
59. An inhibitor of TANGO1-mediated recruitment of ER-Golgi intermediate compartment (ERGIC) membranes, wherein the inhibitor is comprised of one or more inhibitors selected from the group consisting of a polypeptide, a small molecule, a nucleic acid, an antibody, and an antibody fragment.
60. The inhibitor of clause 59, wherein the inhibitor binds to a NRZ tether protein selected from the group consisting of NBAS, RINT1, and ZW10.
61. The inhibitor of clause 59 or 60, wherein the inhibitor binds to a region within SEQ ID NO: 27, SEQ ID NO: 28, or SEQ ID NO: 29.
62. The inhibitor of any one of clauses 59 to 61, wherein the inhibitor prevents the interaction between TANGO1 and one or more proteins selected from the group consisting of NBAS, RINT1, and ZW10.
63. The inhibitor of any one of clauses 59 to 62, wherein the inhibitor comprises a polypeptide having at least 50%, at least 60%, at least 70%, at least 80%, at least 90%, at least 95%, at least 98%, or 100% sequence identity to SEQ ID NO: 1 or SEQ ID NO: 2.
64. The inhibitor of any one of clauses 59 to 63, wherein the inhibitor comprises a polypeptide according to SEQ ID NO: 1.
65. The inhibitor of clause 59, wherein the inhibitor binds to a region within SEQ ID NO: 17.
66. The inhibitor of clause 65, wherein the inhibitor binds to a region within residues 33 to 82 of SEQ ID NO: 17.
67. The inhibitor of clause 65, wherein the inhibitor binds to a region within residues 38 to 77 of SEQ ID NO: 17.
68. The inhibitor of clause 65, wherein the inhibitor binds to a region within residues 41 to 75 of SEQ ID NO: 17.
69. The inhibitor of clause 65, wherein the inhibitor binds to a region within residues 42 to 73 of SEQ ID NO: 17.
70. The inhibitor of clause 65, wherein the inhibitor binds to a region within residues 43 to 72 of SEQ ID NO: 17.
71. The inhibitor of clause 65, wherein the inhibitor binds within the region starting from residue 33, 38, 41, 42, or 43, and ending at residue 82, 77, 75, 73, or 72, of SEQ ID NO: 17.
72. The inhibitor of any one of clauses 59 to 71, wherein the inhibitor further comprises one or more signal sequences.
73. The inhibitor of clause 72, wherein the one or more signal sequences comprise a polypeptide selected from the group consisting of SEQ ID NO: 13, SEQ ID NO: 30, SEQ ID NO: 31, SEQ ID NO: 32, SEQ ID NO: 33, SEQ ID NO: 34, SEQ ID NO: 35, and SEQ ID NO: 36.
74. The inhibitor of clause72, wherein the one or more signal sequences encode one or more polypeptides comprising selected from the group consisting of SEQ ID NO: 13, SEQ ID NO: 30, SEQ ID NO: 31, SEQ ID NO: 32, SEQ ID NO: 33, SEQ ID NO: 34, SEQ ID NO: 35, and SEQ ID NO: 36.
75. The inhibitor of clause 73 or 74, wherein the signal sequence is appended to the polypeptide.
76. The inhibitor of clause 75, wherein the signal sequence is appended to the N' terminus of the polypeptide.
77. The inhibitor of clause 75, wherein the signal sequence is appended to the C' terminus of the polypeptide.
77a. The inhibitor of any one of clauses 59 to 77, wherein the nucleic acid is RNA.
78. The inhibitor of clause 77a, wherein the RNA is selected from the group consisting of shRNA, siRNA, miRNA, antisense oligonucleotides, RNA aptamers, modified RNA, native RNA, and combinations thereof.
79. The antisense oligonucleotide of clause 78, wherein the antisense oligonucleotide is
   (a) between 7 and 15 nucleotides long, and wherein the oligonucleotide is a locked nucleic acid;
   (b) between 18 and 25 nucleotides long, and wherein the oligonucleotide is a 2'-O-methyl phosphorothioate ribonucleic acid; or
   (c) between 18 and 25 nucleotides long, and wherein the oligonucleotide is a 2'-O-methoxyethyl-modified phosphorothioate ribonucleic acid.
80. The antisense oligonucleotide of clause 78 or 79, wherein at least one cytosine residue within the oligonucleotide is methylated.
81. The inhibitor of any one of clauses 59 to 80, wherein the antibody is selected from the group consisting of a monoclonal, polyclonal, bispecific, single-domain, and chimeric antibody.
82. The inhibitor of any one of the preceding clauses, in combination with one or more further inhibitors.
83. The inhibitor of clause 82, wherein the one or more further inhibitors comprise inhibitors selected from the group consisting of a polypeptide, a small molecule, a nucleic acid, an antibody, and an antibody fragment.
84. A combination of at least two inhibitors of TANGO1 activity, TANGO1 heterodimerisation, and/or TANGO1-mediated recruitment of ER-Golgi intermediate compartment (ERGIC) membranes, wherein the first inhibitor is an inhibitor according to any one of clauses 1 to 81, and the second inhibitor is a different inhibitor according to any one of clauses 1 to 81.
85. A vector encoding the inhibitor of any one of clauses 1 to 81, or the inhibitors of any one of clauses 82 to 84.
86. The vector of clause 85, wherein the vector is selected from the group consisting of plasmid, adenovirus, adeno-associated virus (AAV), lentivrus (LV), cytomegalovirus (CMV), herpes simplex virus (HSV), vesicular stomatitis virus (VSV), and baculovirus.
87. An expression cassette encoding the inhibitor of any one of clauses 1 to 81, or the inhibitors of any one of clauses 82 to 84.
88. An mRNA encoding the inhibitor of any one of clauses 1 to 81, or the inhibitors of any one of clauses 82 to 84.
89. The mRNA of clause 88, wherein the mRNA is comprised within an RNA vaccine, a circular RNA, a synthetic RNA, an RNA nanostructure, or an RNA/DNA nanostructure.
90. A delivery vehicle comprising the inhibitor of any one of clauses 1 to 81, the inhibitors of any one of clauses 82 to 84, the vector of clauses 85 or 86, the expression cassette of clause 87, or the mRNA of clauses 88 or 89, wherein the delivery vehicle is selected from the group consisting of a nanoparticle, a vesicle, a nanovesicle, a lipid nanoparticle, a lipid nanovesicle, a liposome, and an exosome.
91. A pharmaceutical composition comprising the inhibitor of any one of clauses 1 to 81, the inhibitors of any one of clauses 82 to 84, the vector of clauses 85 or 86, the expression cassette of clause 87, or the mRNA of clauses 88 or 89, or the delivery vehicle of clause 90, further comprising a pharmaceutically acceptable excipient.
92. The pharmaceutical composition of clause 91, wherein the composition is formulated for topical administration.
93. The pharmaceutical composition of clause 91 or 92, wherein the pharmaceutical composition is provided as a lotion, soap, ointment, eye drop, patch, dressing, gauze, cream, gel, dermal spray, solution, or foam.
94. The pharmaceutical composition of any one of clauses 91 to 93 wherein the pharmaceutically acceptable excipient is one or more selected from the group consisting of glycerin, propylene glycol, dimethicone, squalane, shea butter, cocoa butter, carbomer, xanthan gum, cetyl alcohol, stearyl alcohol, cellulose derivatives, methylparaben, propylparaben, phenoxyethanol, propylene glycol, dimethyl sulfoxide, vitamin e, ascorbic acid, fragrance oils, colorants, polysorbate 80, sodium lauryl sulfate, acrylates/c10-30 alkyl acrylate crosspolymer, polyvinylpyrrolidone, hydroxyethyl urea, ethanol, and isopropyl alcohol.
95. A therapeutic agent comprising the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94, for use in medicine.
96. A therapeutic agent comprising the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 95, for use in a method of treating a fibrotic disease or disorder in a subject, the method comprising administering the therapeutic agent to a subject in need thereof.
97. The therapeutic agent for use according to clause 96, wherein the fibrotic disease or disorder is one or more selected from the group consisting of a scarring, sclerosing and fibrosing disease or disorder.
98 The therapeutic agent for use according to clause 96 or 97, wherein the disease or disorder is selected from the group consisting of scleroderma, local scleroderma, dermatofibrosis lenticularis disseminata, morphea, keloids, peyronie's disease, nephrogenic systemic fibrosis, scarring, idiopathic pulmonary fibrosis, non-idiopathic pulmonary fibrosis, chronic kidney disease, systemic scleroderma skin fibrosis, hypertrophic scars, liver fibrosis, and cardiovascular disease.
99. A therapeutic agent comprising the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94, for use in a method of treating or managing a wound.
100. The therapeutic agent for use according to clause 99, wherein the therapeutic agent is applied to the wound.
101. The therapeutic agent for use according to clause 98 to 100, wherein the method further comprises administering the therapeutic agent to the subject in need thereof topically, orally, intravenously, intramuscularly, subcutaneously, transdermally, inhalationally, and/or rectally.
102. The therapeutic agent of any one of clauses 99 to 101, wherein the therapeutic agent prevents or reduces scarring.
103. A therapeutic agent comprising the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94, for use in a method of preventing and/or reducing scarring.
104. The therapeutic agent for use according to any one of clause 98 to 103, wherein the method comprises topical administration of the therapeutic agent.
105. The therapeutic agent for use according to clause 104, wherein the topical administration is five times daily, four times daily, three times daily, twice daily, daily, every two days, every three days, every four days, every five days, every six days, weekly, fortnightly, or monthly.
106. The inhibitor of any one of clauses 1 to 81, or the inhibitors of any one of clauses 82 to 84, for use in a method of treating a fibrotic disease or disorder in a subject, the method comprising administering the therapeutic agent to a subject in need thereof.
107. The inhibitor of any one of clauses 1 to 81, or the inhibitors of any one of clauses 82 to 84, for use in a method of treating a fibrosing, sclerosing, and/or scarring disease or disorder in a subject, the method comprising administering the therapeutic agent to a subject in need thereof.
108. The inhibitor for use according to clause 106 or 107, wherein the disease or disorder is selected from the group consisting of scleroderma, dermatofibrosis lenticularis disseminata, morphea, keloids, peyronie's disease, nephrogenic systemic fibrosis, and scarring.
109. The inhibitor of any one of clauses 1 to 81, or the inhibitors of any one of clauses 82 to 84, for use in a method of treating or managing a wound.
110. The inhibitor for use according to clause 109, wherein the inhibitor is applied to the wound.
111. The inhibitor of clause 109 or 110, wherein the therapeutic agent prevents or reduces scarring.
112. The inhibitor of any one of clauses 1 to 81, or the inhibitors of any one of clauses 82 to 84, for use in a method of preventing and/or reducing scarring.
113. The inhibitor for use according to any one of clauses 106 to 112, wherein the method comprises topical administration of the therapeutic agent.
114. The inhibitor for use according to clause 113, wherein the topical administration is five times daily, four times daily, three times daily, twice daily, daily, every two days, every three days, every four days, every five days, every six days, weekly, fortnightly, or monthly.
115. A method of treating a fibrosing, sclerosing, and/or scarring disease or disorder, the method comprising administering to a subject in need thereof a therapeutic agent selected from the group consisting of the inhibitor of any one of clauses 1 to 81, the inhibitors of any one of clauses 78 to 80, the vector of clauses 81 or 82, the expression cassette of clause 83, or the mRNA of clauses 84 or 85, the delivery vehicle of clause 86, or the pharmaceutical composition of any one of clauses 87 to 90.
116. A method of treating a fibrotic disease or disorder, the method comprising administering to a subject in need thereof a therapeutic agent selected from the group consisting of the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94.
117. The method of clauses 115 or 116, wherein the disease or disorder is selected from the group consisting of scleroderma, local scleroderma, dermatofibrosis lenticularis disseminata, morphea, keloids, peyronie's disease, nephrogenic systemic fibrosis, scarring, idiopathic pulmonary fibrosis, non-idiopathic pulmonary fibrosis, chronic kidney disease, systemic scleroderma skin fibrosis, hypertrophic scars, liver fibrosis, and cardiovascular disease.
118. A method of treating or managing a wound, the method comprising administering to a subject in need thereof a therapeutic agent selected from the group consisting of the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94.
119. The method of clause 118, wherein the therapeutic agent is applied to the wound.
120. A method preventing and/or reducing scarring, the method comprising administering to a subject in need thereof a therapeutic agent selected from the group consisting of the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94.
121. A cosmetic method for treating scarring or a skin blemish, the method comprising administering to a subject in need thereof an agent selected from the group consisting of the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94.
122. The method of any one of clauses 115 to 121, wherein the method comprises topical administration of the therapeutic agent.
123. The method of clause 122, wherein the topical administration is five times daily, four times daily, three times daily, twice daily, daily, every two days, every three days, every four days, every five days, every six days, weekly, fortnightly, or monthly.
124. The method of any one of clauses 115 to 123, wherein the method further comprises administering the therapeutic agent to the subject in need thereof orally, intravenously, intramuscularly, subcutaneously, transdermally, inhalationally, rectally, and/or topically.
125. A method of reducing collagen and/or ECM protein export from the endoplasmic reticulum of a cell, the method comprising contacting the cell with the inhibitor of any one of clauses 1 to 81, the inhibitors of any one of clauses 82 to 84, the vector of clauses 85 or 86, the expression cassette of clause 87, or the mRNA of clauses 88 or 89, the delivery vehicle of clause 90, or the pharmaceutical composition of any one of clauses 91 to 94.
126. A method of screening for an inhibitor of TANGO1 heterodimerisation, the method comprising
   (a) providing one or more target regions for inhibition of TANGO1 heterodimerisation;
   (b) providing a library of candidate inhibitors;
   (c) contacting the candidate inhibitors with the one or more target regions; and
   (d) assessing the ability of candidate inhibitors to bind to the one or more target regions.
127. The method of clause 126, wherein the one or more target regions are within regions selected from the group consisting of SEQ ID NOs: 14 to 26.
128. The method of clause 127, wherein the one or more target regions are within regions selected from the group consisting of SEQ ID NOs: 17, 18, and 23.
129. The method of clause 128, wherein the one or more target regions are selected from the group consisting of SEQ ID NOs: 3, 5, 7, and 9.
130. A method of screening for an inhibitor of TANGO1-mediated recruitment of ERGIC membranes, the method comprising
   (a) providing one or more target regions for inhibition of TANGO1-mediated recruitment of ERGIC membranes;
   (b) providing a library of candidate inhibitors;
   (c) contacting the candidate inhibitors with the one or more target regions; and
   (d) assessing the ability of candidate inhibitors to bind to the one or more target regions.
131. The method of clause 130, wherein the one or more target regions are within regions selected from the group consisting of SEQ ID NOs: 14 to 20, and 27 to 29.
132. The method of clause 130 or 131, wherein the one or more target regions comprise SEQ ID NO: 1.
133. A method of screening for an inhibitor of TANGO1 activity, the method comprising
   (a) providing one or more target regions for inhibition of TANGO1 activity, wherein the target regions are selected from a group comprising SEQ ID NOs: 14 to 29;
   (b) providing a library of candidate inhibitors, optionally wherein the candidate inhibitors comprise candidate inhibitors selected from the group consisting of SEQ ID NOs: 1 to 10;
   (c) contacting the candidate inhibitors with the one or more target regions; and
   (d) assessing the ability of candidate inhibitors to bind to the one or more target regions.
134. The method of any one of clauses 126 to 133, wherein the library of candidate inhibitors is a small molecule, antibody, peptide, nucleotide, or phage display library.
135. The method of any one of clauses 126 to 134, wherein the one or more target regions each comprise a signal or indicator molecule.
136. The method of clause 135, wherein the signal or indicator molecule is selected from the group consisting of a fluorophore, luminescent proteins, bioluminescent proteins, chemiluminescent tags, radio-tags, phosphorescent tags, quantum dots, nanoparticles, upconversion nanoparticles, fluorescent dyes, Raman labels, and cellular barcodes.
137. The method of any one of clauses 126 to 136, wherein step (d) is assessed through the use of a signal or indicator which emits a signal when a candidate inhibitor successfully binds to the one or more target regions.
138. The method of clause 137, wherein the signal emitted is selected from the group consisting of fluorescence, luminescence, bioluminescence, phosphorescence, chemiluminescence, and radioactive decay.
139. The method of any one of clauses 126 to 138, wherein any or all of the steps are carried out *in silico.*

## Claims

1. An inhibitor of TANGO1 activity, wherein the inhibitor is comprised of one or more inhibitors selected from the group consisting of a polypeptide, a small molecule, a nucleic acid, an antibody, and an antibody fragment.

2. The inhibitor of claim 1, wherein the inhibitor is an inhibitor of TANGO1 heterodimerisation and/or homodimerisation.

3. The inhibitor of claim 1 or 2, wherein the inhibitor binds to one or more target proteins selected from the group consisting of TANGO1, TALI, and cTAGE5, optionally wherein the inhibitor binds to a cytoplasmic portion of TANGO1, TALI, and/or cTAGE5.

4. The inhibitor of any one of the preceding claims, wherein the inhibitor binds to a region within SEQ ID NO: 17, SEQ ID NO: 18, SEQ ID NO: 22, and/or SEQ ID NO: 23.

5. The inhibitor of any one of the preceding claims, wherein the inhibitor comprises one or more polypeptides having at least 50%, at least 60%, at least 70%, at least 80%, at least 90%, at least 95%, at least 98%, or 100% sequence identity to the sequences selected from the group consisting of SEQ ID NO: 1 to SEQ ID NO: 10.

6. The inhibitor of claim 1, wherein the inhibitor is an inhibitor of TANGO1-mediated recruitment of ER-Golgi intermediate compartment (ERGIC) membranes.

7. The inhibitor of claim 6, wherein the inhibitor binds to a NRZ tether protein selected from the group consisting of NBAS, RINT1, and ZW10.

8. The inhibitor of claim 6 or 7, wherein the inhibitor binds to a region within SEQ ID NO: 27, SEQ ID NO: 28, or SEQ ID NO: 29.

9. The inhibitor of any one of claims 6 to 8, wherein the inhibitor comprises a polypeptide having at least 50%, at least 60%, at least 70%, at least 80%, at least 90%, at least 95%, at least 98%, or 100% sequence identity to SEQ ID NO: 1 or SEQ ID NO: 2.

10. The inhibitor of any preceding claim, wherein the inhibitor further comprises one or more signal sequences, optionally wherein the one or more signal sequences comprise a polypeptide selected from the group consisting of SEQ ID NO: 13, SEQ ID NO: 30, SEQ ID NO: 31, SEQ ID NO: 32, SEQ ID NO: 33, SEQ ID NO: 34, SEQ ID NO: 35, and SEQ ID NO: 36.

11. A pharmaceutical composition comprising the inhibitor of any one of claims 1 to 10, further comprising a pharmaceutically acceptable excipient.

12. A therapeutic agent comprising the inhibitor of any one of claims 1 to 10, or the pharmaceutical composition of claim 11, for use in medicine.

13. The therapeutic agent of claim 12, for use in a method of treating a fibrotic disease or disorder in a subject, the method comprising administering the therapeutic agent to a subject in need thereof.

14. The therapeutic agent for use according to claim 13, wherein the fibrotic disease or disorder is one or more selected from the group consisting of a scarring, sclerosing and fibrosing disease or disorder, optionally wherein the disease or disorder is selected from the group consisting of scleroderma, local scleroderma, dermatofibrosis lenticularis disseminata, morphea, keloids, peyronie's disease, nephrogenic systemic fibrosis, scarring, idiopathic pulmonary fibrosis, non-idiopathic pulmonary fibrosis, chronic kidney disease, systemic scleroderma skin fibrosis, hypertrophic scars, liver fibrosis, and cardiovascular disease.

15. A method of screening for an inhibitor of TANGO1 activity, the method comprising
(a) providing one or more target regions for inhibition of TANGO1 activity, wherein the target regions are selected from a group comprising SEQ ID NOs: 14 to 29;
(b) providing a library of candidate inhibitors, optionally wherein the candidate inhibitors comprise candidate inhibitors selected from the group consisting of SEQ ID NOs: 1 to 10;
(c) contacting the candidate inhibitors with the one or more target regions; and
(d) assessing the ability of candidate inhibitors to bind to the one or more target regions.
